# EUROPÄISCHE PATENTSCHRIFT

(11) **EP 0 937 724 B1**
(45) Veröffentlichungstag und Bekanntmachung des Hinweises auf die Patenterteilung: **26.10.2005**
(21) Anmeldenummer: 99102917.4
(22) Anmeldetag: 13.02.1999
(51) Int. Cl.: C07D 471/06, C09B 5/62, C09B 67/22

(54) **Perylenverbindungen und ihre Verwendung als Pigmentdispergatoren**
Perylene compounds and their use as pigment dispersants
Composés de pérylène et leur utilisation comme agents dispersants de pigments

(30) Priorität: 21.02.1998 DE 19807422; 06.08.1998 DE 19835757
(43) Veröffentlichungstag der Anmeldung: 25.08.1999
(73) Patentinhaber: Clariant GmbH, 65929 Frankfurt am Main (DE)
(72) Erfinder: Weber, Joachim Dr., 65929 Frankfurt (DE); Urban, Manfred, 65205 Wiesbaden (DE); Dietz, Erwin Dr., 61462 Königstein (DE)

(56) Entgegenhaltungen:
- EP-A- 0 046 164
- EP-A- 0 302 973
- EP-A- 0 864 613
- DD-A- 299 733
- DE-A- 3 926 563
- DUFF J M ET AL: "Molecular Structure and Photoactivity in Perylene Tetracarboxylic Diimides" CHEM. FUNCT. DYES, PROC. INT. SYMP., 2ND 1992,1993, Seiten 564-571, XP002106704
- DATABASE WPI Section Ch, Week 9324 Derwent Publications Ltd., London, GB; Class A97, AN 93-191685 XP002106705 -& JP 05 117541 A (TOYO INK MFG CO) , 14. Mai 1993

## Beschreibung

Die vorliegende Erfindung betrifft neue Pigmentdispergatoren und Pigmentzubereitungen mit verbesserten coloristischen und rheologischen Eigenschaften sowie deren Herstellung und Verwendung zum Pigmentieren von hochmolekularen Materialien.

Pigmentzubereitungen sind Kombinationen von Pigmenten und strukturell zu Pigmenten analogen Pigmentdispergatoren, die mit spezifisch wirksamen Gruppen substituiert sind. Sie werden den Pigmenten zugesetzt, um die Dispergierung in den Anwendungsmedien, insbesondere in Lacken, zu erleichtern und um die rheologischen und coloristischen Eigenschaften der Pigmente zu verbessern. Die Viskosität der hochpigmentierten Lackkonzentrate (Millbase) wird erniedrigt und die Flockung der Pigmentteilchen wird vermindert. Dadurch kann beispielsweise die Transparenz erhöht werden. Dies ist insbesondere bei Metallicpigmenten erwünscht.

Es gibt eine Vielzahl von Vorschlägen zur Verbesserung der rheologischen und coloristischen Eigenschaften von organischen Pigmenten durch Zusatz von Pigmentdispergatoren, die jedoch nicht immer zum erhofften Resultat führen.

Die EP-B-0 321 919 beschreibt die Herstellung von Pigmentzubereitungen durch Mischen der Basispigmente mit methylenimidazolylgruppenhaltigen Pigmentderivaten. Auf dem Gebiet der Perylenpigmente werden Pigmentzubereitungen erhalten, deren coloristische Eigenschaften nicht mehr den heutigen Anforderungen genügen.

Die DE-A-3 106 906 beschreibt die Herstellung von sulfonamidgruppenhaltigen Pigmentdispergatoren. Die dort beschriebenen Pigmentdispergatoren auf der Basis von Perylenverbindungen besitzen jedoch erhebliche coloristische und rheologische Mängel.

JP 05 117 541 offenbart Pigmentzubereitungen aus C.I. Pigment Red 179 und einen Perylendispergator mit Sulfonamidgruppen.

Die US-A-4 762 569 beschreibt die Herstellung von Pigmentzubereitungen auf der Basis von symmetrischen Perylen-3,4,9,10-tetracarbonsäurediimiden. Diese Pigmentzubereitungen sind nur für den Einsatz in lösemittelhaltigen Systemen geeignet. Sie erfüllen nicht alle Anforderungen hinsichtlich der rheologischen und coloristischen Eigenschaften, die an Pigmentzubereitungen gestellt werden. Die coloristischen Eigenschaften sind insbesondere bei hohen Pigmentdispergatorgehalten nicht mehr ausreichend und in vielen Fällen ist ein deutlicher Glanzverlust und eine deutliche Farbtonabweichung feststellbar. Außerdem besitzen diese Pigmentdispergatoren eine ungenügende Lösemittel- und Überlackierechtheit, wodurch ihr universeller Einsatz sehr eingeschränkt ist. Desweiteren können Unverträglichkeiten mit dem Bindemittel in manchen Lacksystemen beobachtet werden, die prohibitiv für den Einsatz sind.

Es bestand daher die Aufgabe, Pigmentzubereitungen zur Verfügung zu stellen, die die genannten Nachteile des Standes der Technik im Hinblick auf Coloristik, Rheologie und universelle Anwendbarkeit überwinden.

Es wurde gefunden, daß die Aufgabe überraschenderweise durch Pigmentzubereitungen gelöst wird, die neben den Basispigmenten ein oder mehrere bestimmte Perylen-3,4,9,10-tetracarbonsäurediimide enthalten.

Gegenstand der Erfindung sind Perylenverbindungen der allgemeinen Formel (I), 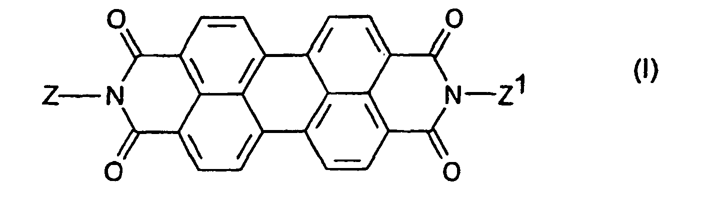 worin
- Z¹: ein Rest der Formel (Ia) ist,

- [X- Y]_{q} - [X¹ - Y¹]ᵣ - [X² - NH]ₛ H (Ia)

worin
X, X¹ und X² gleich oder verschieden sind und einen verzweigten oder unverzweigten C₂-C₆-Alkylenrest oder einen C₅-C₇-Cycloalkylenrest bedeuten, der durch 1 bis 4 C₁-C₄-Alkylreste, Hydroxyreste, Hydroxyalkylreste mit 1 bis 4 Kohlenstoffatomen und/oder durch 1 bis 2 weitere C₅-C₇-Cycloalkylreste substituiert sein kann;
Y eine NH-, -O-, 1,4-Piperazindiyl- oder N(C₁-C₆-alkyl)-Gruppe, Y¹ eine -NH- oder -O-Gruppe, bedeuten;
q eine Zahl von 1 bis 6, vorzugsweise 1, 2, 3 oder 4;
r und s unabhängig voneinander eine Zahl von 0 bis 6, vorzugsweise 0, 1 oder 2,
wobei r und s nicht gleichzeitig Null sind; und wobei s von Null verschieden ist, wenn
Y¹ die Bedeutung -O- hat;
Z die Bedeutung Z¹, Z² oder Z³ hat, worin Z² ein Rest der Formel (Ib) ist,

- [X - O]_{q1} - [X¹ - O]_{q} H (Ib)

worin
q 1 eine Zahl von 0 bis 6, vorzugsweise 0, 1, 2, 3 oder 4;
und Z³ Wasserstoff, Hydroxy, Amino oder C₁-C₈-Alkyl ist, wobei die Alkylgruppe durch 1 bis 4 Substituenten aus der Gruppe Cl, Br, CN, OH,C₆H₅, Carbamoyl, C₁-C₄-Acyl, C₁-C₄-Alkoxy und NR²R³ substituiert sein kann, oder perfluoriert oder teilfluoriert ist, wobei
R² und R³ unabhängig voneinander ein Wasserstoffatom, eine substituierte oder unsubstituierte, oder teil- oder perfluorierte Alkylgruppe mit 1 bis 20 Kohlenstoffatomen, oder eine substituierte oder unsubstituierte, oder teil- oder perfluorierte Alkenylgruppe mit 2 bis 20 Kohlenstoffatomen darstellen, wobei die Substituenten Hydroxy, Phenyl, Cyano, Chlor, Brom, C₂-C₄-Acyl oder C₁-C₄-Alkoxy sein können, oder R² und R³ zusammen mit dem N-Atom einen Imidazolyl-, Piperidinyl-, Morpholinyl-, Pipecolinyl-, Pyrrolyl-, Pyrrolidinyl-, Pyrazolyl-, oder Piperazinyl-Ring bilden.

Je nach Auswahl der Reste Z und Z¹ werden symmetrische oder asymmetrische Pigmentdispergatoren der Formel (I) erhalten. Asymmetrische Pigmentdispergatoren der Formel (I) sind auch solche, die unterschiedliche Reste Z¹ haben.

Bevorzugt sind Perylenverbindungen der Formel (I), worin X, X¹ und X² ein C₂-C₄-Alkylenrest oder Cyclohexylen sind.

Von besonderem Interesse sind beispielsweise Perylenverbindungen der Formel (I), worin Z¹ eine der Bedeutungen -(CH₂)₃-NH-(CH₂)₃-NH₂, -(CH₂)₂-NH-(CH₂)₂-NH₂, -(CH₂)₃-NH-(CH₂)₂-NH-(CH₂)₃-NH₂, 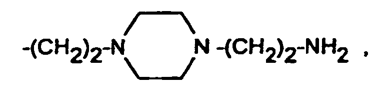 -(CH₂)₃-N(CH₃)-(CH₂)₃-NH₂, -(CH₂)₃-O-(CH₂)₂-O-(CH₂)₃-NH₂, -(CH₂)₃-O-(CH₂)₃-O-(CH₂)₃-NH₂, -(CH₂)₂-NH-(CH₂)₃-NH₂, -(CH₂)₃-NH-(CH₂)₂-NH₂, -(CH₂)₂-NH-(CH₂)₂-NH-(CH₂)₂-NH₂, -(CH₂-CH₂-NH)₄-H, -(CH₂-CH₂-NH)₅-H oder -(CH₂)₃-O-(CH₂)₄-O-(CH₂)₃-NH₂ hat.

Von Interesse sind beispielsweise Perylenverbindungen der Formel (I), worin Z² eine der Bedeutungen -CH(CH₂OH)₂ oder -(CH₂)₂-O-(CH₂)₂OH hat

Von Interesse sind beispielsweise Perylenverbindungen der Formel (I), worin Z³ Wasserstoff, Benzyl, C₁-C₆-Alkyl oder ein durch 1 bis 2 Substituenten aus der Gruppe Hydroxy, Acetyl, Methoxy und Ethoxy substituiertes C₂-C₆-Alkyl, besonders bevorzugt Wasserstoff, Methyl, Ethyl, Propyl, Butyl, Benzyl, Hydroxyethyl, Hydroxypropyl oder Methoxypropyl ist.

Weiterhin von Interesse sind Perylenverbindungen der Formel (I), worin Z³ ein Rest der Gruppe -(CH₂)ₙ-NR²R³ ist, worin n eine Zahl von 1 bis 6, vorzugsweise 2 bis 4, und
R² und R³ unabhängig voneinander ein Wasserstoffatom, eine C₁-C₆-Alkylgruppe, eine durch 1 bis 2 Substituenten aus der Gruppe Hydroxy, Acetyl, Methoxy, Ethoxy, Chlor und Brom substituierte C₁-C₆-Alkylgruppe, oder R² und R³ zusammen mit dem angrenzenden N-Atom einen Imidazolyl-, Piperidinyl-, Morpholinyl-, Pipecolinyl-, Pyrrolyl-, Pyrrolidinyl-, Pyrazolyl- oder Piperazinyl-Ring bilden.

Von besonderem Interesse sind in diesem Zusammenhang Perylenverbindungen, worin
n die Zahl 2 oder 3, und
R² und R³ jeweils eine Methyl- oder Ethylgruppe, oder R² und R³ zusammen mit dem angrenzenden Stickstoffatom einen Imidazolyl-, Piperazinyl- oder Morpholinylrest bilden.

Gegenstand der vorliegenden Erfindung ist auch ein Verfahren zur Herstellung der Perylenverbindungen der allgemeinen Formel (I) durch Umsetzung von Perylen-3,4,9,10-tetracarbonsäuremonoanhydridmonoimiden der allgemeinen Formel (IIa) 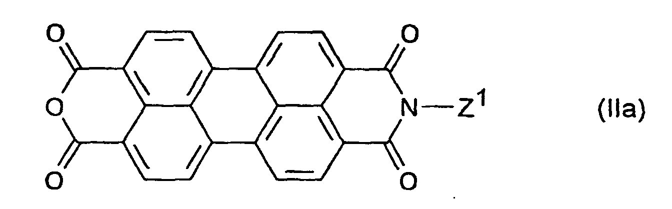 mit einem oder mehreren, vorzugsweise 1 oder 2 Aminen der Formeln (IIIa) oder (IIIb)

Z - NH₂ (IIIa)

Z¹ - NH₂ (IIIb);

oder durch Umsetzung von Perylen-3,4,9,10-tetracarbonsäuremonoanhydridmonoimiden der allgemeinen Formel (IIb) 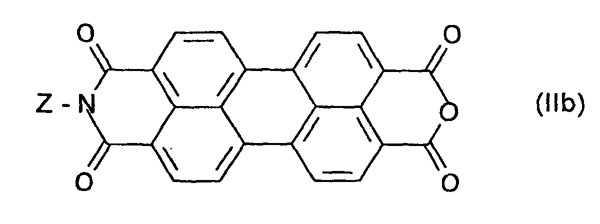 mit einem oder mehreren, vorzugsweise 1 oder 2, Aminen der Formel (IIIb); oder durch Umsetzung von Perylen-3,4,9,10-tetracarbonsäure-dianhydrid der Formel (IIc) 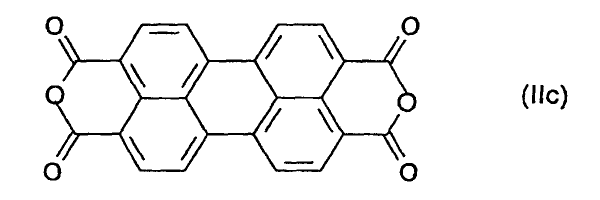 mit einem oder mehreren, vorzugsweise 1 oder 2, Aminen der Formel (IIIb).

Die Anhydridgruppen in den Formeln (IIa), (IIb) und (IIc) können auch als Dicarbonsäuren vorliegen.

Die erfindungsgemäße Umsetzung kann in wäßrigem, organischem oder wäßrig-organischem Medium bei Temperaturen von 0 bis 200°C, vorzugsweise 20 bis 180°C, durchgeführt werden. Als organisches Medium kommen inerte organische Lösemittel, bevorzugt solche, deren Siedepunkt über dem von Wasser liegt, in Betracht, beispielsweise DMSO, Chlorbenzol, Dichlorbenzole, Trichlorbenzole, höhersiedende Alkohole, Carbonamide und höhersiedende Ether. Auch die Amine der Formeln (IIIa) oder (IIIb) können gleichzeitig als Lösemittel dienen. Der pH-Wert des wäßrigen oder wäßrig-organischen Mediums kann sauer, neutral oder alkalisch sein, vorzugsweise zwischen pH 3 bis 14. Es können auch verschiedene Amine gleichzeitig eingesetzt werden, um Mischungen von Perylenverbindungen der Formel (I) herzustellen.

Die Kondensation wird besonders bevorzugt in wäßriger Lösung unter alkalischen pH-Bedingungen bei Temperaturen im Bereich zwischen 50 und 180°C durchgeführt. Zweckmäßig werden hierbei die Amine im Überschuß eingesetzt, zweckmäßig in einem bis zu 8-fachen, vorzugsweise bis zu einem 4-fachen molaren Überschuß. Die Isolierung der gebildeten Verfahrensprodukte der Formel (I) aus dem Reaktionsgemisch erfolgt vorzugsweise durch Filtration.

Als Amine der allgemeinen Formel (IIIa) können beispielsweise Ammoniak, Methylamin, Ethylamin, Propylamin, Butylamin, β-Hydroxyethylamin, N,N-Dimethylamino-propylendiamin, N,N-Diethylamino-propylendiamin, N,N-Dimethylamino-ethylen-diamin, N-Methylaminopropylendiamin, β- oder γ-Hydroxypropylamin, 2-(2-Aminoethoxy)ethanol, Hydroxylamin oder Hydrazin eingesetzt werden.

Als Amine der allgemeinen Formel (IIIb) können beispielsweise Diethylentriamin, Triethylentetramin, Tetraethylenpentamin, Pentaethylenhexamin, Dipropylentriamin, N,N-Bis-(3-aminopropyl)methylamin, Tripropylentetramin, 3-(2-Aminoethyl)-aminopropylamin, N,N'-Bis-(3-aminopropyl)ethylendiamin, 4,7-Dioxadecan-1,10-diamin, 4,9-Dioxadodecan-1,12-diamin oder 1,4-Bis-(3-aminopropoxy)-butan eingesetzt werden.

Die erfindungsgemäßen Perylenverbindungen der Formel (I) dienen beispielsweise zur Herstellung von Pigmentzubereitungen.

Gegenstand der vorliegenden Erfindung sind auch Pigmentzubereitungen, gekennzeichnet durch einen Gehalt an
a) mindestens einem organischen Basispigment aus der gruppe C.I. Pigment Red 123 (C.I. No. 71145), C.I. Pigment Red 149 (C.I. No. 71137), C.I. Pigment Red 178 (C.I. No. 71 155), C.I. Pigment Red 179 (C.I. No. 71 130), C.I. Pigment Red 190 (C.I. 71 140), C.I. Pigment Red 224 (C.I. No. 71 127), sowie aus der gruppe der Perinon-, Phthalocyanin-, Dioxazin-, Chinacridon-, Azo-,Anthrachinon-, Aminoanthrachinon-,Thioindigo-, Diketopyrrolopyrrol-, Flavanthron-, Indanthron-, Isoindolin-, Isoindolinon-, Anthrapyrimidin-, Pyranthron-, Chinophthalon-, Isoviolanthron-,Triarylcarbonium-, Carbon Black- (Ruß), Anthanthronpigmente und
b) mindestens einem Pigmentdispergator der allgemeinen Formel (IV), 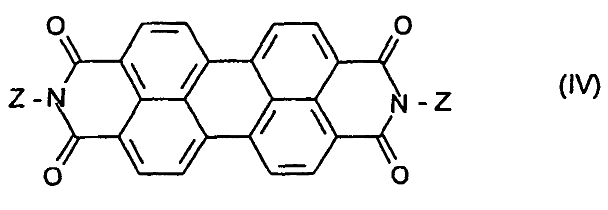
worin die beiden Reste Z gleich oder verschieden sind und die vorstehenden Bedeutungen haben, mit der Maßgabe, daß beide Reste Z nicht gleichzeitig Z³ bedeuten,
ausgenommen eine Pigmentzubereitung, gekennzeichnet durch einen Gehalt an
a) mindestens einem organischen Basispigment aus der Klasse der Perylen-, Perinon-, Chinacridon-, Azo-, Benzimidazolon-, Anthrachinon- und AnthanthronPigmente und
b) mindestens einem Pigmentdispergator der Formel (IV), worin einer der beiden Reste Z die Bedeutung C₁-C₈-Alkyl, das durch 1 bis 4 Hydroxygruppen substituiert ist, hat, und der andere der beiden Reste Z die Bedeutung -(CH₂)ₙ-NR²R³ hat, worin n eine Zahl von 1 bis 6 ist, und R² und R³ die in Anspruch 1 genannten Bedeutungen haben.

Die Herstellverfahren der Pigmentdispergatoren der Formel (IV) können analog denen der Perylenverbindungen der Formel (I) sein.

Unter Basispigment werden organische Pigmente oder Mischungen organischer Pigmente verstanden, die auch als übliche Pigmentzubereitungen vorliegen können. Es kann auch mehr als ein Basispigment eingesetzt werden.

Bevorzugte Basispigmente im Sinne der vorliegenden Erfindung sind beispielsweise auch C.I. Pigment Violet 29 (C.I. No. 71 129); C.I. Pigment Orange 43 (C.I. No. 71 105), C.I. Pigment Red 194 (C.I. No. 71 100); C.I. Pigment Violet 19 (C.I. No. 73 900), C.I. Pigment Red 122 (C.I. No. 73 915); C.I. Pigment Red 209 (C.I. No. 73 905); C.I. Pigment Yellow 147, C.I. Pigment Red 168 (C.I. No. 59 300); C.I. Pigment Yellow 120 (C.I. No. 11 783); C.I. Pigment Yellow 151 (C.I. No. 13 980), C.I. Pigment Brown 25 (C.I. No. 12 510), C.I. Pigment Violet 32 (C.I. No. 12 517), C.I. Pigment Red 170 (C.I. No. 12 475), C.I. Pigment Orange 38 (C.I. No. 12 367), C.I. Pigment Red 188 (C.I. No. 12 467), C.I. Pigment Red 187 (C.I. No. 12 486), C.I. Pigment Orange 34 (C.I. No. 21 115), C.I. Pigment Orange 13 (C.I. No. 21 110), C.I. Pigment Red 9 (C.I. No. 12 460), C.I. Pigment Red 2 (C.I. No. 12 310), C.I. Pigment Red 112 (C.I. No. 12 340), C.I. Pigment Red 7 (C.I. No. 12 420), C.I. Pigment Red 210 (C.I. No. 12 477), C. I. Pigment Red 12 (C.I. No. 12 385), C.I. Pigment Red 202 (C.I. No. 73 907), C.I. Pigment Blue 60 (C.I. No. 69 800), C.I. Pigment Green 7 (C.I. No. 74 260),C.I. Pigment Green 36 (C.I. No. 74 265), C.I. Pigment Blue 15:1, 15:2, 15:3, 15:4 und 15 (C.I. No. 74 160), C.I. Pigment Blue 56 (C.I. No. 42 800), C.I. Pigment Blue 61 (C.I. No. 42 765:1), C.I. Pigment Violet 37 (C.I. No. 51 345), C.I. Pigment Red 177 (C.I. No. 65 300), C.I. Pigment Red 254 (C.I. No. 56 110), C.I. Pigment Red 255, 264, C.I. Pigment Orange 73, C.I. Pigment Violet 23 (C.I. No. 51 319), C.I. Pigment Red 88 (C.I. No. 73 312).

Die Menge der Pigmentdispergatoren der Formel (IV) in den erfindungsgemäßen Pigmentzubereitungen kann, soweit die angestrebte Pigmentqualität nicht negativ beeinflußt wird, in weiten Grenzen schwanken, doch kommt im allgemeinen ein Gehalt von 0,5 bis 40 Gew.-%, insbesondere von 1 bis 20 Gew.-%, an mindestens einem der Pigmentdispergatoren der Formel (IV), bezogen auf das Gewicht des Basispigments, in Betracht.

Die erfindungsgemäßen Pigmentzubereitungen können neben dem Basispigment und dem Pigmentdispergator der Formel (IV) noch weitere übliche Zusatzstoffe enthalten, wie beispielsweise Füllstoffe, Stellmittel, oberflächenaktive Mittel, Harze, Entschäumer, Antistaubmittel, Extender, Farbmittel, Konservierungsmittel, Trocknungsverzögerungsmittel. Besagte Farbmittel werden vorzugsweise zum Nuancieren eingesetzt.

Von Interesse sind weiterhin Pigmentzubereitungen, enthaltend
a) mindestens ein organisches Basispigment,
   b1) einen Pigmentdispergator der Formel (IX) 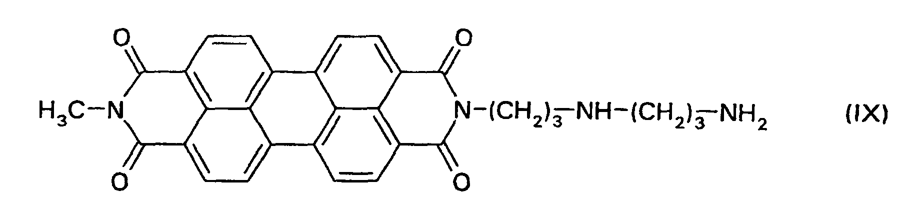 und
   b2) einen Pigmentdispergator der Formel (X)

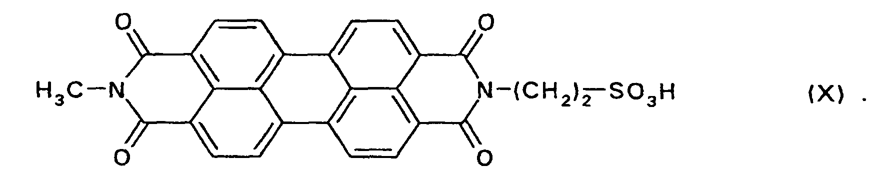

Die Mengenverhältnisse zwischen dem Pigmentdispergator gemäß b1) und b2) können zwischen 1:10 und 10:1 Gewichtsteilen liegen, bevorzugt zwischen 1:5 und 5:1 Gewichtsteilen, insbesondere zwischen 1:3 und 3:1 Gewichtsteilen.

Bevorzugte Pigmentzubereitungen im Sinne der vorliegenden Erfindung bestehen im wesentlichen aus
a) 40 bis 99,5 Gew.-%, vorzugsweise 60 bis 99 Gew.-%, mindestens eines Basispigments,
b) 0,5 bis 40 Gew.-%, vorzugsweise 1 bis 20 Gew.-%, mindestens eines, vorzugsweise 1, 2 oder 3, Pigmentdispergators der Formel (IV),
c) 0 bis 20 Gew.-%, vorzugsweise 0,1 bis 15 Gew.-% an oberflächenaktiven Mitteln und
d) 0 bis 20 Gew.-%, vorzugsweise 0,1 bis 10 Gew.-% an anderen üblichen Zusatzstoffen,
wobei die Anteile der jeweiligen Komponenten auf das Gesamtgewicht der Zubereitung (100 Gew.-%) bezogen sind.

Als oberflächenaktive Mittel kommen übliche anionische, kationische oder nichtionische Tenside in Betracht, beispielsweise anionaktive Substanzen wie Fettsäuretauride, Fettsäure-N-methyltauride, Fettsäureisethionate, Alkylbenzolsulfonate, Alkylnaphthalinsulfonate, Alkylphenolpolyglykolethersulfate und Fettalkoholpolyglykolethersulfate; Fettsäuren, z.B. Palmitin-, Stearin- und Ölsäure; Seifen, z.B. Alkalisalze von Fettsäuren, Naphthensäuren und Harzsäuren, z.B. Abietinsäure, alkalilöslische Harze, z.B. kolophoniummodifizierte Maleinatharze; kationaktive Substanzen, wie quarternäre Ammoniumsalze, Fettaminoxethylate, Fettaminpolyglykolether und Fettamine; nichtionogene Substanzen wie Fettalkoholpolyglykolether, Fettalkoholpolyglykolester und Alkylphenolpolyglykolether.

Bei den erfindungsgemäßen Pigmentzubereitungen handelt es sich in der Regel um feste Systeme von rieselfähiger, pulverförmiger Beschaffenheit oder um Granulate.

Der erfindungsgemäß erzielbare Dispergiereffekt beruht vermutlich auf einer Modifizierung der Oberflächenstruktur der Basispigmente mit den Pigmentdispergatoren der Formel (IV). So sind in einer Reihe von Fällen die Wirksamkeit der Pigmentdispergatoren der Formel (IV) und die Qualität der damit erzeugten Pigmentzubereitungen abhängig vom Zeitpunkt der Zugabe des Dispergators im Herstellungsprozess des Basispigments. Auch die Art und Weise der Applikation des Pigmentdispergators der Formel (IV) kann einen Einfluß haben.

Die Wirksamkeit der Pigmentdispergatoren der Formel (IV) kann auch von der Teilchengröße und Teilchenform der Pigmentdispergatoren der Formel (IV) sowie vom Umfang der belegbaren Pigmentoberfläche abhängen. Es kann ebenso vorteilhaft sein, den Pigmentdispergator der Formel (IV) erst im in Aussicht genommenen Anwendungsmedium dem Basispigment direkt zuzufügen. Die jeweilige optimale Konzentration des Pigmentdispergators der Formel (IV) muß durch orientierende Vorversuche ermittelt werden, da die Verbesserung der Eigenschaften der Basispigmente nicht immer linear mit der Pigmentdispergatormenge einhergeht.

Die erfindungsgemäßen Pigmentzubereitungen können Mischungen von einem oder mehreren, vorzugsweise 1 oder 2, Basispigmenten mit einem oder mehreren, vorzugsweise 1, 2 oder 3, der Pigmentdispergatoren der Formel (IV) sein.

Gegenstand der Erfindung ist auch ein Verfahren zur Herstellung einer erfindungsgemäßen Pigmentzubereitung, dadurch gekennzeichnet, daß man den oder die Pigmentdispergator(en) der Formel (IV) und das oder die Basispigment(e) an einem beliebigen Zeitpunkt ihres Herstellungsprozesses aufeinander einwirken läßt.

Der Herstellungsprozeß eines organischen Pigments umfaßt dessen Synthese, gegebenenfalls Feinverteilung, z.B. durch Mahlung oder Umfällen, gegebenenfalls Lösemittelfinish, sowie die Isolierung als Preßkuchen oder als trockenes Granulat oder Pulver. Beispielsweise können die Pigmentdispergatoren der Formel (IV) vor oder während der Pigmentsynthese, vor oder während eines Feinverteilungsprozesses oder einer anschließenden Lösemittelbehandlung (Finish) zugegeben werden. Dabei können Temperaturen von 0 bis 200 °C auftreten. Selbstverständlich kann der Pigmentdispergator der Formel (IV) auch in Teilportionen zu unterschiedlichen Zeiten zugegeben werden.

Die Zugabe der Pigmentdispergatoren der Formel (IV) im Rahmen eines Feinverteilungsprozesses erfolgt beispielsweise im Verlauf einer Trockenmahlung eines Rohpigments mit oder ohne zusätzliche Mahlhilfsmittel auf einer Roll- oder Schwingmühle, oder im Zuge einer Naßmahlung eines Rohpigments in wäßrigem, wäßrig-organischem oder organischem Mahlmedium, beispielsweise auf einer Perlmühle.

Gleichfalls bewährt hat sich die Zugabe der Pigmentdispergatoren der Formel (IV) vor oder während eines Finishs für das Basispigment in wäßrigem, wäßrigalkalischem, wäßrig-organischem oder organischem Medium.

Die Pigmentdispergatoren der Formel (IV) können auch dem wasserfeuchten Pigmentpreßkuchen vor der Trocknung zugesetzt und eingearbeitet werden, wobei der Pigmentdispergator der Formel (IV) selbst ebenfalls als Preßkuchen vorliegen kann.

Es ist weiterhin möglich, Trockenmischungen von pulverförmigen Pigmentdispergatoren der Formel (IV) mit dem Pulver oder Granulat eines oder mehrerer Basispigmente vorzunehmen.

Weiterhin ist es möglich, den Pigmentdispergator der Formel (IV) und ein Perylenpigment durch Umsetzung mit demselben Amin der Formel (IIIa) oder (IIIb) als Mischung zu synthetisieren.

Die erfindungsgemäßen Perylenverbindungen der Formel (I) zeichnen sich durch hervorragende Echtheiten, wie z.B. Lösemittelechtheiten aus, und sind für den Einsatz sowohl in lösemittelhaltigen als auch in wäßrigen Systemen geeignet.

Die nach der vorliegenden Erfindung erhältlichen Pigmentzubereitungen zeichnen sich aus durch ihre hervorragenden coloristischen und rheologischen Eigenschaften, insbesondere durch eine hervorragende Rheologie, hohe Flockungsstabilität, hohe Transparanz, leichte Dispergierbarkeit, gutes Glanzverhalten, hohe Farbstärke, einwandfreie Überlackier- und Lösemittelechtheiten sowie sehr gute Wetterechtheit. Sie sind für den Einsatz sowohl in lösemittelhaltigen als auch in wäßrigen Systemen geeignet.

Die erfindungsgemäß hergestellten Pigmentzubereitungen lassen sich zum Pigmentieren (Einfärben) von hochmolekularen organischen Materialien natürlicher oder synthetischer Herkunft einsetzen.

Hochmolekulare organische Materialien, die mit den genannten Pigmentzubereitungen pigmentiert werden können, sind beispielsweise Celluloseether und -ester, wie Ethylcellulose, Nitrocellulose, Celluloseacetat oder Cellulosebutyrat, natürliche Harze oder Kunstharze, wie Polymerisationsharze oder Kondensationsharze, z.B. Aminoplaste, insbesondere Harnstoff- und Melaminformaldehydharze, Alkydharze, Acrylharze, Phenoplaste, Polycarbonate, Polyolefine, wie Polystyrol, Polyvinylchlorid, Polyethylen, Polypropylen, Polyacrylnitril, Polyacrylsäureester, Polyamide, Polyurethane oder Polyester, Gummi, Casein, Silikon und Silikonharze, einzeln oder in Mischungen.

Dabei spielt es keine Rolle, ob die erwähnten hochmolekularen organischen Verbindungen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemäß erhaltenen Pigmentzubereitungen als Blend oder in Form von Präparationen oder Dispersionen zu benutzen. Bezogen auf das zu pigmentierende, hochmolekulare organische Material setzt man die erfindungsgemäßen Pigmentzubereitungen in einer Menge von vorzugsweise 0,1 bis 10 Gew.-% ein.

Die erfindungsgemäßen Perylenverbindungen der Formel (I) und die erfindungsgemäßen Pigmentzubereitungen sind geeignet als Farbmittel in elektrophotographischen Tonern und Entwicklern, wie z.B. Ein- oder Zweikomponentenpulvertonem (auch Ein- oder Zweikomponenten-Entwickler genannt), Magnettoner, Flüssigtoner, Polymerisationstoner sowie Spezialtoner (Lit: L.B. Schein, "Electrophotography and Development Physics"; Springer Series in Electrophysics 14, Springer Verlag, 2nd edition, 1992).

Typische Tonerbindemittel sind Polymerisations-, Polyadditions- und Polykondensationsharze, wie Styrol-, Styrolacrylat-, Styrolbutadien-, Acrylat-, Polyester-, Phenol-Epoxidharze, Polysulfone, Polyurethane, einzeln oder in Kombination, sowie Polyethylen und Polypropylen, die noch weitere Inhaltsstoffe, wie Ladungssteuermittel, Wachse oder Fließhilfsmittel, enthalten können oder im Nachhinein mit diesen Zusätzen modifiziert werden.

Desweiteren sind die erfindungsgemäßen Perylenverbindungen der Formel (I) und die erfindungsgemäßen Pigmentzubereitungen geeignet als Farbmittel in Pulver und Pulverlacken, insbesondere in triboelektrisch oder elektrokinetisch versprühbaren Pulverlacken, die zur Oberflächenbeschichtung von Gegenständen aus beispielsweise Metall, Holz, Kunststoff, Glas, Keramik, Beton, Textilmaterial, Papier oder Kautschuk zur Anwendung kommen (J.F. Hughes, "Electrostatics Powder Coating" Research Studies, John Wiley & Sons, 1984).

Als Pulverlackharze werden typischerweise Epoxidharze, carboxyl- und hydroxylgruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harzsystem) sind beispielsweise Säureanhydride, Imidazole sowie Dicyandiamid und deren Abkömmlinge, verkappte Isocyanate, Bisacylurethane, Phenol- und Melaminharze, Triglycidylisocyanurate, Oxazoline und Dicarbonsäuren.

Außerdem sind die erfindungsgemäßen Perylenverbindungen der Formel (I) und die erfindungsgemäßen Pigmentzubereitungen als Farbmittel in Ink-Jet Tinten auf wäßriger und nichtwäßriger Basis sowie in solchen Tinten, die nach dem hot-melt-Verfahren arbeiten, geeignet.

Es ist auch möglich, daß erst im Anwendungsmedium der Pigmentdispergator der Formel (IV) dem Basispigment, oder umgekehrt, zugegeben wird. Gegenstand der Erfindung ist daher auch eine Pigmentpräparation, bestehend im wesentlichen aus dem besagten Basispigment, dem besagten Pigmentdispergator der Formel (IV), dem besagten hochmolekularen organischen Material, insbesondere Lack, gegebenenfalls oberflächenaktiven Mittel und/oder weiteren üblichen Zusatzstoffen. Die Gesamtmenge an Basispigment plus Pigmentdispergator der Formel (IV) ist vorzugsweise 1 bis 10 Gew.-%, bezogen auf das Gesamtgewicht der Pigmentpräparation.

Zur Beurteilung der Eigenschaften der nach der Erfindung hergestellten Pigmentzubereitungen auf dem Lacksektor wurden aus der Vielzahl der bekannten Lacke ein aromatenhaltiger Alkydmelaminharzlack (AM) auf Basis eines mittelöligen Alkydharzes und eines butanolveretherten Melaminharzes, ein Polyesterlack (PE) auf Basis von Celluloseacetobutyrat und eines Melaminharzes, ein High-Solid-Acrylharzeinbrennlack auf Basis einer nichtwäßrigen Dispersion (HS) sowie ein wäßriger Lack auf Polyurethanbasis (PUR) ausgewählt.

Die Bestimmung der Farbstärke und des Farbtons erfolgte nach DIN 55986.

Die Rheologie des Mahlguts nach der Dispergierung (Millbase-Rheologie) wurde visuell anhand der folgenden fünfstufigen Skala bewertet
- 5: dünnflüssig
- 4: flüssig
- 3: dickflüssig
- 2: leicht gestockt
- 1: gestockt

Nach dem Verdünnen des Mahlguts auf die Pigmentendkonzentration wurde die Viskosität mit dem Viskospatel nach Rossmann, Typ 301 der Firma Erichsen beurteilt.

Glanzmessungen erfolgten an Folienaufgüssen unter einem Winkel von 20° nach DIN 67530 (ASTMD 523) mit dem "multigloss"-Glanzmeßgerät der Firma Byk-Mallinckrodt. Die Bestimmung der Lösemittelechtheit erfolgte nach DIN 55976. Die Bestimmung der Überlackierechtheit erfolgte nach DIN 53221. Die Bestimmung der Kristallphase der Pigmente und Pigmentzubereitungen erfolgte durch Röntgenspektroskopie. Die Aufnahme der Röntgenspektren erfolgte mit Cu Kα-Strahlung.

In den nachfolgenden Beispielen beziehen sich Teile jeweils auf Gewichtsteile und Prozente jeweils auf Gewichtsprozente der so beschriebenen Substanzen.

### Beispiel 1

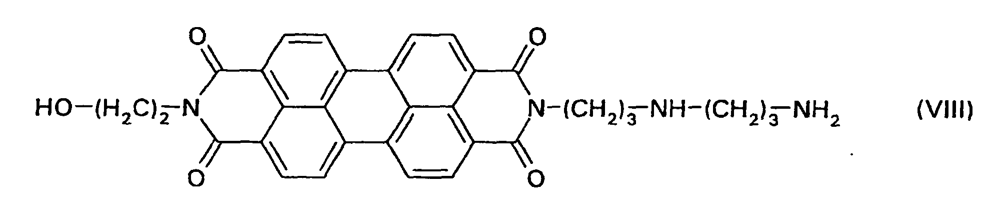

In einem Autoklaven werden 300 Teile Wasser vorgelegt, 22,7 Teile Perylen-3,4,9,10-tetracarbonsäuremonoanhydridmonohydroxyethylimid eingetragen und 26,2 Teile Dipropylentriamin zugegeben. Anschließend wird unter Druck auf 150 °C erhitzt und 5 Stunden bei 150 °C gerührt. Nach dem Abkühlen auf 25 °C wird der Pigmentdispergator abgesaugt und mit Wasser neutral gewaschen.

Man erhält 143,2 Teile Presskuchen Pigmentdispergator 19,1 %ig. Ein Teil wird für die Analyse bei 80°C getrocknet.

| | | |
|---|---|---|
| Analyse | Ber. | 70.1 % C |
| | Gef. | 70.0 % C |

Das ¹H-NMR-Spektrum stimmt mit der oben angegebenen Strukturformel überein. δ (D₂SO₄) 8,65; 6,1; 5,65; 5,16; 4,5; 4,16; 2,97; 2,81; 2,09 und 1,76 ppm.

Die Lösemittelechtheit des Pigmentdispergators ist sehr gut.

### Beispiel 1a

In einem Rührgefäß werden 363,5 Teile Wasser vorgelegt und unter Rühren 108,9 Teile Filterkuchen Perylen-3,4,9,10-tetracarbonsäuredianhydrid 20,7 %ig eingetragen. Zu dieser Suspension werden 2,4 Teile einer handelsüblichen 50 %igen wäßrigen Harzseife gegeben und 6,2 Teile Presskuchen Pigmentdispergator 19,1 %ig der Formel VIII, hergestellt gemäß Beispiel 1, eingetragen. Nach Abkühlung auf 0 bis 5°C, werden während 10 Minuten 40,8 Teile einer 40 %igen wäßrigen Monomethylaminlösung zugetropft. Es wird noch 15 Minuten bei 0 bis 5°C gerührt. Dann wird eine Lösung aus 12,8 Teilen Calciumchlorid *2H₂O in 42,5 Teilen Wasser zugetropft und 1 Stunde bei 0 bis 5°C gerührt. Die Suspension wird auf 80°C erhitzt und 2 Stunden bei 80°C gerührt. Nach dem Abkühlen auf 50°C wird bei dieser Temperatur Essigsäure zugetropft, bis ein pH-Wert von 8 erreicht ist. Die erhaltene Pigmentzubereitung wird abgesaugt, mit Wasser chlorionenfrei gewaschen und bei 80°C im Umluftschrank getrocknet. Man erhält 27 Teile Pigmentzubereitung.

Die Pigmentzubereitung liefert im HS-Lack transparente und farbstarke Lackierungen. Die Rheologie wird mit 4 bewertet und die Glanzmessung ergibt den Wert 73. Die Rheologie des PUR-Lacks der Pigmentzubereitung wird mit 4 bewertet.

### Beispiel 2

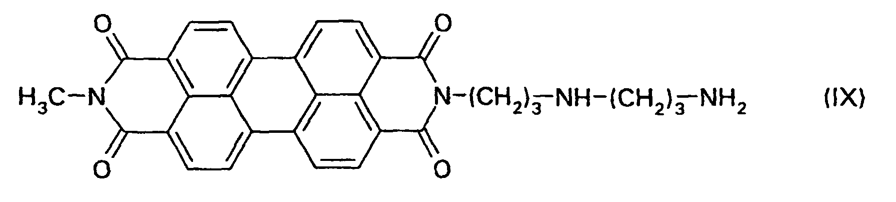

In einem Autoklaven werden 450 Teile Wasser vorgelegt, 37 Teile Perylen-3,4,9,10-tetracarbonsäuremonoanhydridmonomethylimid eingetragen und 47,1 Teile Dipropylentriamin zugegeben. Anschließend wird unter Druck auf 150°C erhitzt und 5 Stunden bei 150°C gerührt. Nach dem Abkühlen auf 25°C wird der Pigmentdispergator abgesaugt und mit Wasser neutral gewaschen. Man erhält 263 Teile Presskuchen Pigmentdispergator 15,5 %ig.

| | | |
|---|---|---|
| Analyse | Ber. | 12.3 % O |
| | Gef. | 13.0 % O |

¹H-NMR (D₂SO₄) δ 8,7; 6,1; 5,7; 4,2; 3,5; 3,0; 2,8; 2,0 und 1,8 ppm.
Die Lösemittelechtheit des Pigmentdispergators ist sehr gut.

### Beispiel 2a

In einem Rührgefäß werden 3000 Teile Wasser vorgelegt und unter Rühren 540 Teile Filterkuchen Perylen-3,4,9,10-tetracarbonsäuredianhydrid 27,8 %ig eingetragen. Zu dieser Suspension werden 16 Teile einer handelsüblichen 50 %igen wäßrigen Harzseife gegeben und, nach Abkühlung auf 0 bis 5 °C, während 10 Minuten 222 Teile einer 45,5 %igen wäßrigen Monomethylaminlösung zugetropft. Es wird 15 Minuten bei 0 bis 5°C gerührt. Während 15 Minuten wird bei 0 bis 5°C eine Lösung aus 84,9 Teilen wasserfreiem Calciumchlorid in 250 Teilen Wasser zugetropft und 1 Stunde bei 0 bis 5°C gerührt. Die Suspension wird auf 80°C erhitzt und 1 Stunde bei 80 °C gerührt. Danach wird eine Suspension aus 8 Teilen Distearyldimethylammoniumchlorid und 350 Teilen Wasser zugetropft und eine Stunde bei 80°C gerührt. Nach dem Abkühlen auf 50°C wird bei dieser Temperatur 98 %ige Ameisensäure zugetropft, bis ein pH-Wert von 7 erreicht ist. Es wird 1/2 Stunde bei 50°C gerührt, das erhaltene Pigment abgesaugt, mit Wasser chlorionenfrei gewaschen und bei 80°C im Umluftschrank getrocknet. Man erhält 172,3 Teile Pigment (C.I. Pigment Red 179).

20 Teile dieses Pigments werden mit 1 Teil Pigmentdispergator der Formel IX, hergestellt gemäß Beispiel 2, mechanisch gemischt.
Man erhält eine Pigmentzubereitung, die im HS-Lack transparente und farbstarke Lackierungen liefert. Die Rheologie wird mit 4 bewertet. Die Metalliclackierung ist farbstark und brillant.

Ohne den Zusatz des Pigmentdispergators sind die Lackierungen deutlich deckender und wesentlich farbschwächer. Die Rheologie wird mit 1 bewertet.

### Beispiel 2b

30 Teile Pigment (C.I. Pigment Blue 15:1) werden mit 1,5 Teilen Pigmentdispergator der Formel IX, hergestellt gemäß Beispiel 2, mechanisch gemischt.
Man erhält eine Pigmentzubereitung, die im AM-Lack transparente und farbstarke Lackierungen liefert. Die Viskosität beträgt 7,0 s. Die Glanzmessung ergibt den Wert 49.

Ohne den Zusatz des Pigmentdispergators sind die Lackierungen merklich deckender. Die Viskosität beträgt 10,3 s. Die Glanzmessung ergibt den Wert 30.

### Beispiel 2c

30 Teile Pigment (C.I. Pigment Red 194) werden mit 1,5 Teilen Pigmentdispergator der Formel IX, hergestellt gemäß Beispiel 2, mechanisch gemischt.
Man erhält eine Pigmentzubereitung, die im AM-Lack farbstarke Lackierungen liefert. Die Rheologie wird mit 4 bis 5 bewertet. Die Viskosität beträgt 3,3 s. Die Glanzmessung ergibt den Wert 70.

Ohne den Zusatz des Pigmentdispergators ergibt die Glanzmessung den Wert 60.

### Beispiel 2d

30 Teile Pigment (C.I. Pigment Violet 19) werden mit 1,5 Teilen Pigmentdispergator der Formel IX, hergestellt gemäß Beispiel 2, mechanisch gemischt.
Man erhält eine Pigmentzubereitung, die im AM-Lack transparente und farbstarke Lackierungen mit tiefem Farbton liefert. Die Rheologie wird mit 5 bewertet. Die Viskosität beträgt 5,2 s. Die Überlackierechtheit ist einwandfrei.

Ohne den Zusatz des Pigmentdispergators sind die Lackierungen merklich deckender und deutlich heller.

### Beispiel 2e

30 Teile Pigment (C.I. Pigment Red 168) werden mit 0,75 Teilen Pigmentdispergator der Formel IX, hergestellt gemäß Beispiel 2, mechanisch gemischt.
Man erhält eine Pigmentzubereitung, die im AM-Lack transparente und farbstarke Lackierungen mit tiefem Farbton liefert. Die Rheologie wird mit 4 bis 5 bewertet. Die Viskosität beträgt 3,2 s.

Ohne den Zusatz des Pigmentdispergators sind die Lackierungen merklich deckender und wesentlich heller.

### Beispiel 2f

30 Teile Pigment (C.I. Pigment Violet 23) werden mit 1,5 Teilen Pigmentdispergator der Formel IX, hergestellt gemäß Beispiel 2, mechanisch gemischt.
Man erhält eine Pigmentzubereitung, die im AM-Lack farbstarke Lackierungen liefert. Die Rheologie wird mit 3 bewertet. Dis Viskosität beträgt 4,6 s.

Ohne den Zusatz des Pigmentdispergators beträgt die Viskosität 5,1 s.

### Beispiel 2g

In einem Stahlbehälter, der mit 1400 Teilen Steatitkugeln vom Durchmesser 12 mm als Mahlkörper zu 80 Vol.-% gefüllt ist, werden 30 Teile grobkristallines Perylenrohpigment (C.I. Pigment Red 149) (hergestellt gemäß der DE-PS 1 067 157), 150 Teile wasserfreies Natriumsulfat und 1,6 Teile Pigmentdispergator der Formel IX, hergestellt gemäß Beispiel 2, eingefüllt und auf einer Schwingmühle (Typ Vibratom, Hersteller: Siebtechnik, Mühlheim),
8 Stunden bei 1400 Umdehungen pro Minute, Schwingkreis 4 mm, gemahlen. Anschließend wird das Mahlgut von den Mahlkörpern abgesiebt. Das Mahlgut wird in 1500 Teile Wasser eingetragen und 1 Stunde bei 80 °C gerührt. Danach wird die Pigmentzubereitung abgesaugt, mit Wasser salzfrei gewaschen und bei 80 °C getrocknet. Man erhält 28,6 Teile Pigmentzubereitung.

Die Pigmentzubereitung liefert im AM-Lack farbstarke und transparente Lackierungen. Die Viskosität beträgt 12.9 s.

Ohne den Zusatz des Pigmentdispergators sind die Lackierungen deutlich deckender. Die Viskosität ist so hoch, daß sie mit dem Viskospatel nicht meßbar ist.

### Beispiel 2h

30 Teile Pigment (C.I. Pigment Brown 25) werden mit 1.5 Teilen Pigmentdispergator der Formel IX, hergestellt gemäß Beispiel 2, mechanisch gemischt.
Man erhält eine Pigmentzubereitung, die im AM-Lack farbstarke Lackierungen liefert. Die Viskosität beträgt 6.8s. Die Glanzmessung ergibt den Wert 63.

Ohne den Zusatz des Pigmentdispergators beträgt die Viskosität 8.5 s. Die Glanzmessung ergibt den Wert 59.
Im PE-Lack liefert die Pigmentzubereitung farbstarke und transparente Lackierungen mit einwandfreier Überlackierechtheit. Die Metalliclackierung ist farbstark und brillant.
Ohne Zusatz des Pigmentdispergators sind die Lackierungen deckender.

### Beispiel 2i

### Ringschluß und Hydrolyse:

150 Teile 2,5-Dianilinoterephthalsäure werden unter Rühren bei 80 bis 90°C in 750 Teile Polyphosphorsäure, die >84 % P₂O₅ enthält, eingetragen und 1 Stunde auf 125°C erhitzt, wobei der Ringschluß zum Chinacridon erfolgt. Danach wird das Reaktionsgemisch unter Rühren mit 3375 Teilen Phosphorsäure 13,9 %ig mit einer Temperatur von 80°C hydrolysiert. Es wird 1 Stunde bei 105°C gerührt. Danach wird das Rohpigment abgesaugt und neutral gewaschen.
Man erhält 734 Teile eines 18,0 %igen Rohpigment-Filterkuchens, der überwiegend in der α-Phase vorliegt.

### Phasenumwandlung:

694,5 Teile des Rohpigment-Filterkuchens werden in ein Rührgefäß eingefüllt, 680,5 Teile Wasser, 12,9 Teile Natriumhydroxid (98 %ig) und 375 Teile Isobutanol (100 %ig) zugegeben, auf 150°C unter Druck erhitzt und 5 Stunden bei 150°C gerührt. Nach dem Abkühlen auf 90°C wird das Isobutanol bis 100°C am Übergang azeotrop abdestilliert. Die Suspension wird auf 60°C abgekühlt, das Rohpigment abgesaugt, mit Wasser neutral gewaschen und bei 80°C getrocknet.
Man erhält 115,7 Teile hochkristallines Rohpigment, das in der β-Phase vorliegt.

### Mahlung:

In eine Rührwerkskugelmühle (Hersteller: Draiswerke GmbH, Mannheim), die mit 360 Teilen Zirkonmischoxidperlen vom Durchmesser 0,3-0,4 mm als Mahlkörper gefüllt ist, wird eine Suspension, bestehend aus 77 Teilen Natronlauge 1 %ig, 6,3 Teilen grobkristallinem, unsubstituiertem Chinacridonrohpigment (β-Phase) und 0,32 Teilen Pigmentdispergator der Formel IX, hergestellt gemäß Beispiel 2, eindosiert und mit einer Rührwerksumfangsgeschwindigkeit von 15,6 m/s sowie einer spezifischen Leistungsdichte von 3,1 kW pro Liter Mahlraum 15 Minuten lang bei 25 °C gemahlen. Diese Mahlung wird ein zweites Mal durchgeführt, die beiden Mahlgutsuspensionen vereinigt und von den Mahlkörpern abgesiebt, die Mahlkörper werden mit Wasser abgespült. Anschließend werden die Mahlgutsuspensionen abgesaugt, mit Wasser gewaschen und bei 80°C getrocknet.
Man erhält 11,9 Teile Pigmentzubereitung auf Basis von C.I. Pigment Violet 19, die im AM-Lack transparente und farbstarke Lackierungen mit tiefem Farbton liefert. Die Rheologie wird mit 5 bewertet. Die Viskosität beträgt 3,7 s. Die Überlackierechtheit ist einwandfrei. Ohne den Zusatz des Pigmentdispergators sind die Lackierungen wesentlich heller und deutlich deckender.

### Beispiel 2j

30 Teile Pigment (C.I. Pigment Red 122) werden mit 0,75 Teilen Pigmentdispergator der Formel IX, hergestellt gemäß Beispiel 2, mechanisch gemischt.
Man erhält eine Pigmentzubereitung, die im AM-Lack farbstarke und transparente Lackierungen mit tiefem Frabton liefert. Die Rheologie wird mit 4 bis 5 bewertet. Die Glanzmessung ergibt den Wert 55. Die Überlackierechtheit ist einwandfrei. Ohne den Zusatz des Pigmentdispergators sind die Lackierungen merklich deckender und deutlich heller.

### Beispiel 3

15 Teile Pigment (C.I. Pigment Red 179), hergestellt gemäß Beispiel 2a, werden mit 0,75 Teilen Pigmentdispergator der Formel VIII, hergestellt gemäß Beispiel 1, und mit 0,75 Teilen Pigmentdispergator mit der Formel IX, hergestellt gemäß Beispiel 2, mechanisch gemischt.
Man erhält eine Pigmentzubereitung, deren Lösemittelechtheit sehr gut ist. Im HS-Lack liefert die Pigmentzubereitung transparente und farbstarke Lackierungen. Die Rheologie wird mit 4 bewertet und die Viskosität beträgt 2,0 s. Die Glanzmessung ergibt den Wert 69.

Ohne den Zusatz der beiden Pigmentdispergatoren sind die Lackierungen des HS-Lacks farbschwächer und wesentlich deckender. Die Rheologie wird mit 1 bewertet und die Viskosität ist so hoch, daß sie mit dem Viskospatel nicht meßbar ist. Wegen der starken Flockung ist der Glanz nicht meßbar.

Im PUR-Lack liefert die Pigmentzubereitung transparente Lackierungen, die Rheologie wird mit 4 bewertet.
Ohne den Zusatz der beiden Pigmentdispergatoren sind die Lackierungen des PUR-Lacks merklich deckender. Die Rheologie wird mit 3 bis 4 bewertet.

### Beispiel 4

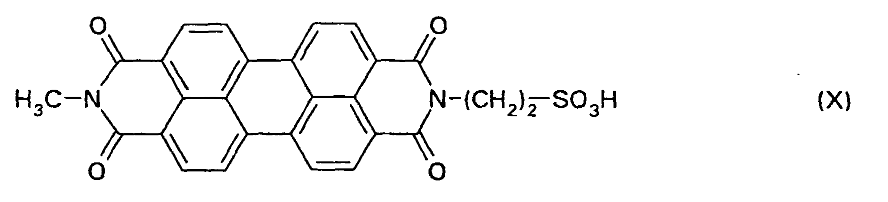

In einem Autoklaven werden 1200 Teile Wasser vorgelegt und 50,1 Teile Taurin eingetragen und gelöst. Durch weitere Zugabe von 26,4 Teilen Kaliumhydroxid (85 %ig) wird in dieser Lösung ein pH-Wert von 9,6 eingestellt. Anschließend trägt man 40,5 Teile Perylen-3,4,9,10-tetracarbonsäuremonoanhydrid-N-monomethylimid ein. Es wird auf 150 °C geheizt und 3 Stunden bei 150 °C gerührt. Nach dem Abkühlen auf 25 °C wird das als K-Salz gebildete Umsetzungsprodukt abgesaugt, mit Wasser gewaschen und bei 80 °C getrocknet. Der erhaltene Rückstand wird bei 25 °C zunächst in 1000 Teilen 100 %ige Schwefelsäure eingebracht und gelöst. Danach werden dieser Lösung noch 850 Teile 50 %ige Schwefelsäure tropfenweise zugefügt, wobei die Temperatur bis 80 °C ansteigen darf. Anschließend läßt man auf 25 °C abkühlen; die so freigesetzte Sulfonsäure wird abgesaugt, mit 78 %iger Schwefelsäure gewaschen, danach mit 31 %iger Salzsäure sulfatfrei gewaschen und im Vakuum bei 80 °C getrocknet.
Man erhält 44,9 Teile Pigmentdispergator der Formel X, die 1,9 % chemisch gebundenes Wasser enthält, entsprechend 44,0 Teile 100 %ig (= 85,9 % d.Th.).

| | |
|---|---|
| Analyse | Unter Berücksichtigung von 1,9 % Wasser |
| Ber. | 63.3 % C |
| Gef. | 63.0 % C |

### Beispiel 4a

100 Teile Pigment (C.I. Pigment Red 179), hergestellt gemäß Beispiel 2a, werden mit 10 Teilen Pigmentdispergator der Formel IX, hergestellt gemäß Beispiel 2, und mit 5 Teilen Pigmentdispergator der Formel X, hergestellt gemäß Beispiel 4, und mit 1 Teil handelsüblichen C.I. Basic Blue 140 mechanisch gemischt.
Man erhält eine Pigmentzubereitung, deren Lösemittelechtheit sehr gut ist. Im HS-Lack liefert die Pigmentzubereitung transparente und farbstarke Lackierungen. Die Rheologie wird mit 4 bis 5 bewertet und die Viskosität beträgt 1,7 s. Die Glanzmessung ergibt den Wert 68. Die Metalliclackierung ist farbstark und brillant.

Ohne den Zusatz der beiden Pigmentdispergatoren sind die Lackierungen farbschwächer und deckender. Die Rheologie wird mit 1 bewertet und die Viskosität ist so hoch, daß sie mit dem Viskospatel nicht meßbar ist. Auch der Glanz ist durch die starke Flockung nicht meßbar. Die Metalliclackierung ist farbschwach und matt.

### Beispiel 5

In einem Rührgefäß werden 2000 Teile Wasser vorgelegt und unter Rühren 90 Teile Perylen-3,4,9,10-tetracarbonsäuredianhydrid und 31 Teile Presskuchen Pigmentdispergator 15,5 %ig der Formel IX, hergestellt gemäß Beispiel 2, eingetragen. Zu dieser Suspension werden 9.6 Teile einer handelsüblichen 50 %igen wäßrigen Harzseife gegeben und, nach Abkühlung auf 0 bis 5°C, während 10 Minuten 163 Teile einer 40 %igen wäßrigen Monomethylaminlösung zugetropft. Es wird noch 15 Minuten bei 0 bis 5°C gerührt. Dann wird eine Lösung aus 51 Teilen Calciumchlorid *2H₂O in 170 Teilen Wasser zugetropft und 1 Stunde bei 0 bis 5°C gerührt. Die Suspension wird auf 80 °C erhitzt und 1 Stunde bei 80 °C gerührt. Danach wird eine Suspension aus 4,8 Teilen Distearyldimethylammoniumchlorid und 280 Teilen Wasser zugetropft und eine Stunde bei 80 °C gerührt. Nach dem Abkühlen auf 50 °C wird bei dieser Temperatur Essigsäure zugetropft, bis ein pH-Wert von 8 erreicht ist. Die erhaltene Pigmentzubereitung wird abgesaugt, mit Wasser chlorionenfrei gewaschen und bei 80 °C im Umluftschrank getrocknet.
Man erhält 106,9 Teile Pigmentzubereitung.

10 Teile der obigen Pigmentzubereitung werden mit 0,42 Teilen Pigmentdispergator der Formel IX, hergestellt gemäß Beispiel 2, und mit 0,42 Teilen Pigmentdispergator der Formel X, hergestellt gemäß Beispiel 4, und mit 0,04 Teilen handelsüblichem C.I. Basic Blue 140 mechanisch gemischt.
Man erhält eine Pigmentzubereitung, deren Lösemittelechtheit sehr gut ist. Im HS-Lack liefert die Pigmentzubereitung transparente und farbstarke Lackierungen. Die Rheologie wird mit 5 bewertet und die Viskosität beträgt 3,0 s. Die Glanzmessung ergibt den Wert 71. Die Metalliclackierung ist farbstark und brillant.

Ohne den Zusatz der beiden Pigmentdispergatoren sind die Lackierungen farbschwächer und deckender. Die Rheologie wird mit 1 bewertet und die Viskosität ist so hoch, daß sie mit dem Viskospatel nicht meßbar ist. Auch der Glanz ist wegen der starken Flockung nicht meßbar. Die Metalliclackierung ist farbschwach und matt.

### Beispiel 6

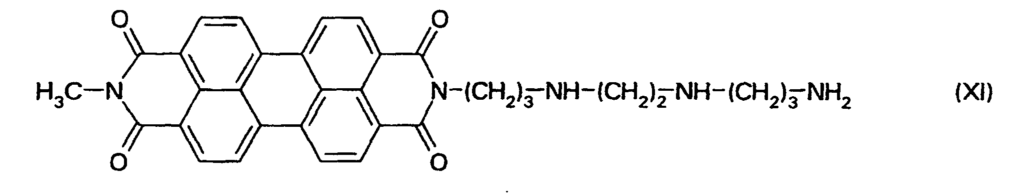

In einem Autoklaven werden 500 Teile Wasser vorgelegt, 40,5 Teile Perylen-3,4,9,10-tetracarbonsäuremonoanhydridmonomethylimid eingetragen und 52,2 Teile N,N'-Bis(3-aminopropyl)ethylendiamin zugegeben. Anschließend wird unter Druck auf 150 °C erhitzt und 5 Stunden bei 150 C gerührt. Nach dem Abkühlen auf 25 °C wird der Pigmentdispergator abgesaugt, mit Wasser neutral gewaschen.
Man erhält 132,2 Teile Presskuchen Pigmentdispergator 34,8 %ig.

| | | |
|---|---|---|
| Analyse | Ber. | 70,6 % C |
| | Gef. | 71,1 % C |

¹H-NMR (D₂SO₄): δ 8,71; 6,32; 5,71; 4,47; 4,17; 3,51; 3,3; 3,0; 2,08 ppm.
Die Lösemittelechtheit des Pigmentdispergators ist sehr gut.

### Beispiel 6a

In einem Rührgefäß werden 368 Teile Wasser vorgelegt und unter Rühren 104,5 Teile Filterkuchen Perylen-3,4,9,10-tetracarbonsäuredianhydrid 21,5 %ig eingetragen. Zu dieser Suspension werden 2,4 Teile einer handelsüblichen 50 %igen wäßrigen Harzseife gegeben und, nach Abkühlung auf 0 bis 5°C, während 10 Minuten 40,8 Teile einer 40 %igen wäßrigen Monomethylaminlösung zugetropft. Es wird noch 15 Minuten bei 0 bis 5°C gerührt. Dann wird eine Lösung aus 12,8 Teilen Calciumchlorid *2H₂O in 42,5 Teilen Wasser zugetropft und 1 Stunde bei 0 bis 5°C gerührt. Die Suspension wird auf 80 °C erhitzt und 1 Stunde bei 80 °C gerührt. Danach wird eine Suspension aus 3,44 Teilen Presskuchen Pigmentdispergator 34.8 %ig der Formel XI, hergestellt gemäß Beipiel 6, und 50 Teilen Wasser zugegeben und eine Stunde bei 80 °C gerührt. Nach dem Abkühlen auf 60 °C wird bei dieser Temperatur Essigsäure zugetropft, bis ein pH-Wert von 8 erreicht ist. Das erhaltene Pigment wird abgesaugt, mit Wasser chlorionenfrei gewaschen und bei 80 °C im Umluftschrank getrocknet. Man erhält 26,3 Teile Pigmentzubereitung.
Die Pigmentzubereitung liefert im HS-Lack transparente und farbstarke Lackierungen. Die Rheologie wird mit 4 bewertet und die Viskosität beträgt 3,1 s. Die Glanzmessung ergibt den Wert 74.

Wenn statt der 5,5 Teile Presskuchen Pigmentdispergator 34,8 %ig der Formel XI, hergestellt gemäß Beipiel 6, eine Suspension aus 1,2 Teilen Distearyldimethylammoniumchlorid und 70 Teilen Wasser zugetropft werden, sind die Lackierungen merklich deckender und wesentlich farbschwächer. Die Rheologie wird mit 1 bewertet und die Viskosität ist so hoch, daß sie mit dem Viskospatel nicht meßbar ist. Auch der Glanz ist durch die starke Flockung nicht meßbar.

### Beispiel 6b

10 Teile Pigmentzubereitung, hergestellt gemäß Beispiel 6a, werden mit 0,45 Teilen Pigmentdispergator der Formel IX, hergestellt gemäß Beispiel 2, mechanisch gemischt.

Die Pigmentzubereitung liefert im HS-Lack transparente und farbstarke Lackierungen. Die Rheologie wird mit 4 bis 5 bewertet. Die Viskosität beträgt 1,2 s. Die Glanzmessung ergibt den Wert 72. Die Metalliclackierung ist farbstark und brillant.

Wenn statt der 5,5 Teile Presskuchen Pigmentdispergator 34,8 %ig der Formel XI, hergestellt gemäß Beipiel 6, eine Suspension aus 1,2 Teilen Distearyldimethylammoniumchlorid und 70 Teilen Wasser zugetropft werden, außerdem keine mechanische Mischung mit 0,45 Teilen Pigmentdispergator mit der Formel IX, hergestellt gemäß Beispiel 2, durchgeführt wird, wird die Rheologie mit 1 bewertet. Die Viskosität ist so hoch, daß sie mit dem Viskospatel nicht meßbar ist. Auch der Glanz ist durch die starke Flockung nicht meßbar. Die Metalliclackierung ist farbschwach und matt.

### Beispiel 7

30 Teile Pigment (C.I. Pigment Red 179), hergestellt gemäß Beispiel 2a, werden mit 1,35 Teilen Pigmentdispergator der Formel IX, hergestellt gemäß Beispiel 2, mechanisch gemischt.
Man erhält eine Pigmentzubereitung deren Lösemittelechtheit sehr gut ist. Im PE3-Lack liefert die Pigmentzubereitung farbstarke Lackierungen mit tiefem Farbton. Die Viskosität beträgt 2,8 s. Die Überlackierechtheit ist einwandfrei.
Ohne Zusatz des Pigmentdispergators sind die Lackierungen heller, die Viskosität beträgt 3,8 s.
Im PUR-Lack liefert die Pigmentzubereitung farbstarke und transparente Lackierungen mit tiefem Farbton, die Rheologie wird mit 3 bis 4 bewertet.
Ohne Zusatz des Pigmentdispergators sind die Lackierungen deckender und heller, die Rheologie wird mit 3 bewertet.

### Beispiel 8

30 Teile Pigment (C.I. Pigment Red 179), hergestellt gemäß Beispiel 2a, werden mit 1,35 Teilen Pigmentdispergator der Formel VIII, hergestellt gemäß Beispiel 1, und mit 1,35 Teilen Pigmentdispergator der Formel IX, hergestellt gemäß Beispiel 2, mechanisch gemischt.
Man erhält eine Pigmentzubereitung, die im PE-Lack farbstarke Lackierungen mit tiefem Farbton liefert. Die Viskosität beträgt 2,6 s. Die Überlackierechtheit ist einwandfrei.
Ohne Zusatz des Pigmentdispergators sind die Lackierungen heller, die Viskosität beträgt 3,8 s.
Im PUR-Lack liefert die Pigmentzubereitung transparente Lackierungen mit tiefem Farbton, die Rheologie wird mit 3 bis 4 bewertet.
Ohne Zusatz des Pigmentdispergators sind die Lackierungen deutlich deckender und wesentlich heller, die Rheologie wird mit 3 bewertet.

### Beispiel 9 (Vergleichsbeispiel)

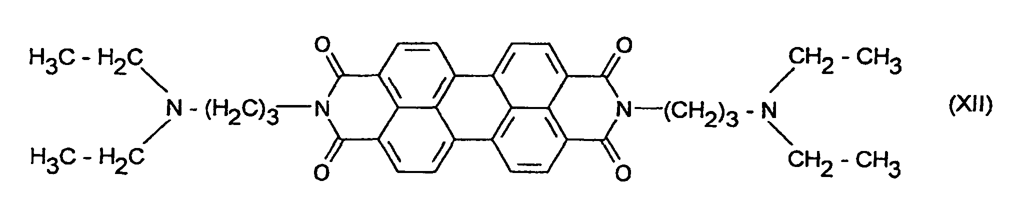

In einem Autoklaven werden 500 Teile Wasser vorgelegt, 189,7 Teile Filterkuchen Perylen-3,4,9,10-tetracarbonsäuredianhydrid 20,7 %ig eingetragen und 52 Teile Diethylaminopropylamin zugegeben. Anschließend wird unter Druck auf 150°C erhitzt und 5 Stunden bei 150 °C gerührt. Nach dem Abkühlen auf 25 °C wird der Pigmentdispergator abgesaugt und mit Wasser neutral gewaschen.
Man erhält 138,2 Teile Presskuchen Pigmentdispergator 45,2 %ig.

| | | |
|---|---|---|
| Analyse | Ber. | 9.1 % N |
| | Gef. | 9.1 % N |

¹H-NMR-Spektrum (D₂SO₄): δ 8,7; 4,1; 2,87; 2,8; 1,9 und 0,9 ppm.
Die Lösemittelechtheit des Pigmentdispergators ist ungenügend. Im Vergleich zu den Lösemittelechtheiten des Pigmentdispergators der Formel VIII, hergestellt gemäß Beispiel 1, des Pigmentdispergators der Formel IX, hergestellt gemäß Beispiel 2, und des Pigmentdispergators der Formel XI, hergestellt gemäß Beispiel 6, ist sie wesentlich schlechter und damit deutlich unterlegen.

### Beispiel 9a (Vergleichsbeispiel)

30 Teile Pigment (C.I. Pigment Red 179), hergestellt gemäß Beispiel 2a, werden mit 1,35 Teilen Pigmentdispergator der Formel XII, hergestellt gemäß Beispiel 9, mechanisch gemischt.
Man erhält eine Pigmentzubereitung, die im HS-Lack Vollton-Lackierungen liefert, die beim Einbrennen nicht mehr vollständig aushärten. Der Glanz kann nicht gemessen werden, da die Folienaufgüsse ebenfalls nicht aushärten. Auch die Metalliclackierung härtet nicht aus. Die Rheologie wird mit 3 bewertet. Im AM-Lack ist die Überlackierechtheit ungenügend.

Dagegen liefert die Pigmentzubereitung des Beispiels 7 (statt 1,35 Teilen Pigmentdispergator der Formel XII, hergestellt gemäß Beispiel 9, wurden 1,35 Teile Pigmentdispergator der Formel IX, hergestellt gemäß Beispiel 2, zur mechanischen Mischung verwendet), im HS-Lack farbstarke und transparente Lackierungen, die beim Einbrennen einwandfrei aushärten. Die Glanzmessung ergibt den Wert 77. Die Metalliclackierung ist farbstark und brillant. Die Rheologie wird mit 4 bewertet.
Auch die Überlackierechtheit im AM-Lack der Lackierung der Pigmentzubereitung des Beispiels 7 ist merklich besser.
Die angeführten Mängel zeigen, daß die Pigmentzubereitung des Beispiels 7 deutlich überlegen ist.

### Beispiel 9b (Vergleichsbeispiel)

20 Teile Pigment (C.I. Pigment Red 179), hergestellt gemäß Beispiel 2a, werden mit 1 Teil Pigmentdispergator der Formel XII, hergestellt gemäß Beispiel 9, mechanisch gemischt.
Man erhält eine Pigmentzubereitung, deren Lösemittelechtheit ungenügend ist. Sie ist wesentlich schlechter im Vergleich zu den Lösemittelechtheiten der Pigmentzubereitungen der Beispiele 3, 4a und 5 und damit deutlich unterlegen.

Im HS-Lack liefert die Pigmentzubereitung Vollton-Lackierungen, die beim Einbrennen nicht mehr vollständig aushärten. Der Glanz kann nicht gemessen werden, da die Folienaufgüsse ebenfalls nicht aushärten. Die Rheologie wird mit 3 bis 4 bewertet.

Dagegen liefert die Pigmentzubereitung des Beispiels 2a (statt 1 Teil Pigmentdispergator der Formel XII, hergestellt gemäß Beispiel 9, wurde 1 Teil Pigmentdispergator der Formel IX, hergestellt gemäß Beispiel 2, zur mechanischen Mischung verwendet), im HS-Lack farbstarke und transparente Lackierungen, die beim Einbrennen einwandfrei aushärten. Die Rheologie wird mit 4 bewertet.

Die angeführten Mängel zeigen, daß die Pigmentzubereitung des Beispiels 2a deutlich überlegen ist.

### Beispiel 10

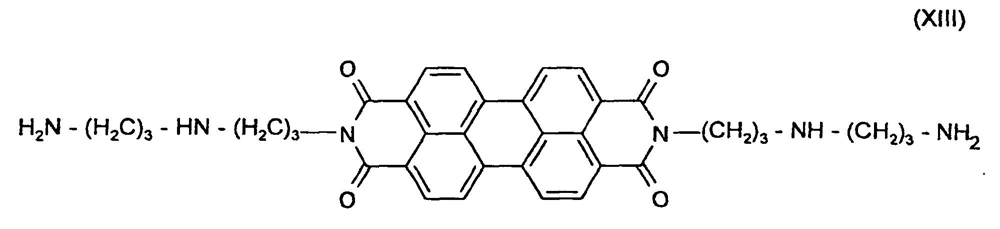

In einem Autoklaven werden 300 Teile Wasser vorgelegt, 189,7 Teile Filterkuchen Perylen-3,4,9,10-tetracarbonsäuredianhydrid 20,7 %ig eingetragen und 104,8 Teile 3,3'-Iminobispropylamin zugegeben. Anschließend wird unter Druck auf 150 °C erhitzt und 5 Stunden bei 150 °C gerührt. Nach dem Abkühlen auf 25 °C wird der Pigmentdispergator abgesaugt und mit Wasser neutral gewaschen.
Man erhält 435,3 Teile Presskuchen Pigmentdispergator 12,5 %ig.

| | | |
|---|---|---|
| Analyse | Ber. | 69.9 % C |
| | Gef. | 68.2 % C |

¹H-NMR-Spektrum (D₂SO₄): δ 8,67; 6,03; 5,65; 4,15; 2,95; 2,8; 1,95 und 1,75 ppm.

### Beispiel 10a

In einem Rührgefäß werden 363.5 Teile Wasser vorgelegt und unter Rühren 108,9 Teile Filterkuchen Perylen-3,4,9,10-tetracarbonsäuredianhydrid 20,7 %ig und 9,6 Teile Presskuchen Pigmentdispergator 12,5 %ig der Formel XIII, hergestellt gemäß Beispiel 10, eingetragen. Zu dieser Suspension werden 2,4 Teile einer handelsüblichen 50 %igen wäßrigen Harzseife gegeben und, nach Abkühlung auf 0 bis 5°C, während 10 Minuten 40,8 Teile einer 40 %igen wäßrigen Monomethylaminlösung zugetropft. Es wird noch 15 Minuten bei 0 bis 5°C gerührt. Dann wird eine Lösung aus 12,8 Teilen Calciumchlorid *2H₂O in 42,5 Teilen Wasser zugetropft und 1 Stunde bei 0 bis 5°C gerührt. Die Suspension wird auf 80°C erhitzt und 1 Stunde bei 80°C gerührt. Danach wird eine Suspension aus 1,2 Teilen Distearyldimethylammoniumchlorid und 70 Teilen Wasser zugetropft und eine Stunde bei 80°C gerührt. Nach dem Abkühlen auf 50°C wird bei dieser Temperatur Essigsäure zugetropft, bis ein pH-Wert von 8 erreicht ist. Die erhaltene Pigmentzubereitung wird abgesaugt, mit Wasser chlorionenfrei gewaschen und bei 80°C im Umluftschrank getrocknet.
Man erhält 27,6 Teile Pigmentzubereitung.
Im HS-Lack liefert die Pigmentzubereitung transparente und farbstarke Lackierungen. Die Rheologie wird mit 3 bis 4 bewertet und die Viskosität beträgt 13,6 s. Die Glanzmessung ergibt den Wert 38.
Ohne den Zusatz des Pigmentdispergators sind die Lackierungen deutlich farbschwächer und merklich deckender. Die Rheologie wird mit 1 bewertet und die Viskosität ist so hoch, daß sie mit dem Viskospatel nicht meßbar ist. Auch der Glanz ist wegen der starken Flockung nicht meßbar.

### Beispiel 11

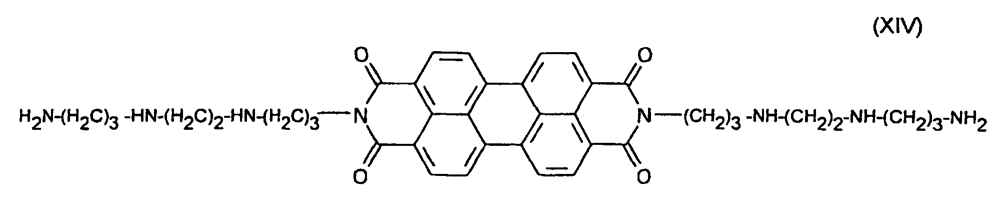

In einem Autoklaven werden 500 Teile Wasser vorgelegt, 181,9 Teile Filterkuchen Perylen-3,4,9,10-tetracarbonsäuredianhydrid 21,5 %ig eingetragen und 69,6 Teile N,N'-Bis(3-aminopropyl)ethylendiamin zugegeben. Anschließend wird unter Druck auf 150°C erhitzt und 5 Stunden bei 150°C gerührt. Nach dem Abkühlen auf 25°C wird der Pigmentdispergator abgesaugt und mit Wasser neutral gewaschen. Man erhält 390,2 Teile Presskuchen Pigmentdispergator 12,9 %ig.

| | | |
|---|---|---|
| Analyse | Ber. | 68.2 % C |
| | Gef. | 67 % C |

¹H-NMR-Spektrum (D₂SO₄): δ 8,7; 6,3;, 5,7; 4,2; 3,3; 3,1; 2,95; 2,85; 2,1 und 1,82 ppm.

### Beispiel 11a

In einem Rührgefäß werden 368 Teile Wasser vorgelegt und unter Rühren 104,5 Teile Filterkuchen Perylen-3,4,9,10-tetracarbonsäuredianhydrid 21,5 %ig eingetragen. Zu dieser Suspension werden 2,4 Teile einer handelsüblichen 50 %igen wäßrigen Harzseife gegeben und 9,3 Teile Presskuchen Pigmentdispergator 12,9 %ig der Formel XIV, hergestellt gemäß Beispiel 11, eingetragen. Nach Abkühlung auf 0 bis 5°C werden während 10 Minuten 40,8 Teile einer 40 %igen wäßrigen Monomethylaminlösung zugetropft. Es wird noch 15 Minuten bei 0 bis 5 °C gerührt. Dann wird eine Lösung aus 12,8 Teilen Calciumchlorid *2H₂O in 42,5 Teilen Wasser zugetropft und 1 Stunde bei 0 bis 5°C gerührt. Die Suspension wird auf 80°C erhitzt und 2 Stunden bei 80°C gerührt. Nach dem Abkühlen auf 60°C wird bei dieser Temperatur Essigsäure zugetropft, bis ein pH-Wert von 8 erreicht ist. Die erhaltene Pigmentzubereitung wird abgesaugt, mit Wasser chlorionenfrei gewaschen und bei 80 °C im Umluftschrank getrocknet.
Man erhält 26 Teile Pigmentzubereitung.
Die Pigmentzubereitung liefert im HS-Lack transparente und farbstarke Lackierungen liefert. Die Rheologie wird mit 3 bis 4 bewertet und die Glanzmessung ergibt den Wert 59. Die Viskosität beträgt 4,8 s.

### Beispiel 11b

10 Teile Pigmentzubereitung, hergestellt gemäß Beispiel 11 a, werden mit 0,45 Teilen Pigmentdispergator der Formel IX, hergestellt gemäß Beispiel 2, mechanisch gemischt.
Man erhält eine Pigmentzubereitung, die im HS-Lack transparente und farbstarke Lackierungen liefert. Die Rheologie wird mit 4 bewertet und die Glanzmessung ergibt den Wert 70. Die Viskosität beträgt 1,2 s.

### Beispiel 12

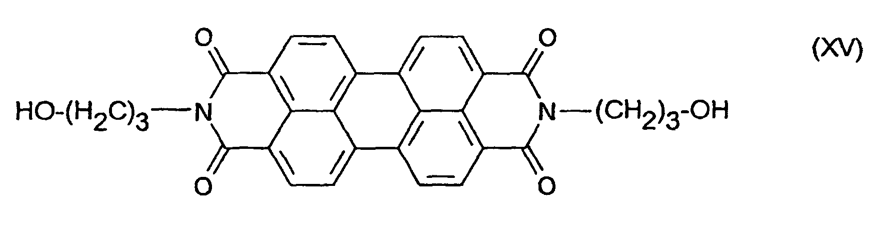

In einem Autoklaven werden 500 Teile Wasser vorgelegt, 181,9 Teile Filterkuchen Perylen-3,4,9,10-tetracarbonsäuredianhydrid 21,5 %ig eingetragen und 30 Teile 3-Amino-1-propanol zugegeben. Anschließend wird unter Druck auf 150 °C erhitzt und 5 Stunden bei 150 °C gerührt. Nach dem Abkühlen auf 25 °C wird der Pigmentdispergator abgesaugt und mit Wasser neutral gewaschen. Man erhält 118 Teile Presskuchen Pigmentdispergator 43,1 %ig.

| | | |
|---|---|---|
| Analyse | Ber. | 71.1 % C |
| | Gef. | 70.8 % C |

¹H-NMR-Spektrum (D₂SO₄): δ 8,67; 4,7; 4;2; 3,9; 2,15 und 2,0 ppm.
Die Lösemittelechtheit des Pigmentdispergators ist sehr gut.

### Beispiel 12a

In einem Rührgefäß werden 368 Teile Wasser vorgelegt und unter Rühren 104,5 Teile Filterkuchen Perylen-3,4,9,10-tetracarbonsäuredianhydrid 21,5 %ig eingetragen. Zu dieser Suspension werden 2,4 Teile einer handelsüblichen 50 %igen wäßrigen Harzseife gegeben und, nach Abkühlung auf 0 bis 5 °C, während 10 Minuten 40,8 Teile einer 40 %igen wäßrigen Monomethylaminlösung zugetropft. Es wird noch 15 Minuten bei 0 bis 5°C gerührt. Dann wird eine Lösung aus 12,8 Teilen Calciumchlorid *2H₂O in 42,5 Teilen Wasser zugetropft und 1 Stunde bei 0 bis 5°C gerührt. Die Suspension wird auf 80 °C erhitzt und 1 Stunde bei 80 °C gerührt. Danach wird eine Suspension aus 2,78 Teilen Presskuchen Pigmentdispergator 43,1 %ig der Formel XV, hergestellt gemäß Beipiel 12, und 50 Teilen Wasser zugegeben und eine Stunde bei 80 °C gerührt. Nach dem Abkühlen auf 60 °C wird bei dieser Temperatur Essigsäure zugetropft, bis ein pH-Wert von 8 erreicht ist. Die erhaltene Pigmentzubereitung wird abgesaugt, mit Wasser chlorionenfrei gewaschen und bei 80 °C im Umluftschrank getrocknet. Die Pigmentzubereitung liefert im HS-Lack transparente und farbstarke Lackierungen. Die Rheologie wird mit 4 bis 5 bewertet.

### Beispiel 12b

10 Teile Pigmentzubereitung, hergestellt gemäß Beispiel 12a, werden mit 0,45 Teilen Pigmentdispergator der Formel IX, hergestellt gemäß Beispiel 2, mechanisch gemischt.
Man erhält eine Pigmentzubereitung, die im HS-Lack transparente und farbstarke Lackierungen liefert. Die Rheologie wird mit 4 bewertet und die Glanzmessung ergibt den Wert 67. Die Viskosität beträgt 1,8 s.

### Beispiel 13

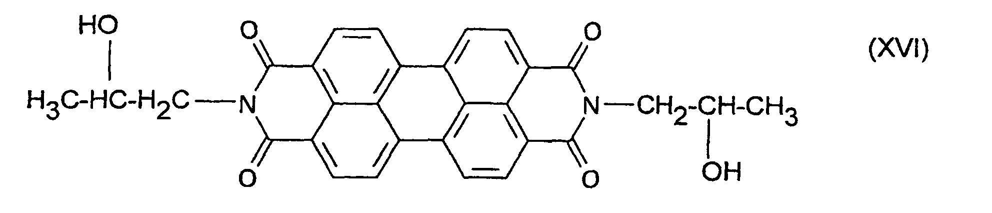

In einem Autoklaven werden 500 Teile Wasser vorgelegt, 181,9 Teile Filterkuchen Perylen-3,4,9,10-tetracarbonsäuredianhydrid 21,5 %ig eingetragen und 30 Teile 1-Amino-2-propanol zugegeben. Anschließend wird unter Druck auf 150 °C erhitzt und 5 Stunden bei 150 °C gerührt. Nach dem Abkühlen auf 25 °C wird der Pigmentdispergator abgesaugt und mit Wasser neutral gewaschen.
Man erhält 139,8 Teile Presskuchen Pigmentdispergator 35,8 %ig.

| | | |
|---|---|---|
| Analyse | Ber. | 71.1 % C |
| | Gef. | 70.9 % C |

¹H-NMR-Spektrum (D₂SO₄): δ 8.67, 5.6, 4.45, 3.9 und 1.5 ppm.
Die Lösemittelechtheit des Pigmentdispergators ist sehr gut.

### Beispiel 13a

15 Teile Pigment (C.I. Pigment Red 179), hergestellt gemäß Beispiel 2a, werden mit 0,75 Teilen Pigmentdispergator der Formel IX, hergestellt gemäß Beispiel 2, und mit 0,75 Teilen Pigmentdispergator der Formel XVI, hergestellt gemäß Beispiel 13, mechanisch gemischt.
Man erhält eine Pigmentzubereitung, die im HS-Lack transparente und farbstarke Lackierungen liefert. Die Rheologie wird mit 4 bewertet und die Glanzmessung ergibt den Wert 73. Die Viskosität beträgt 1,8 s.

### Beispiel 14

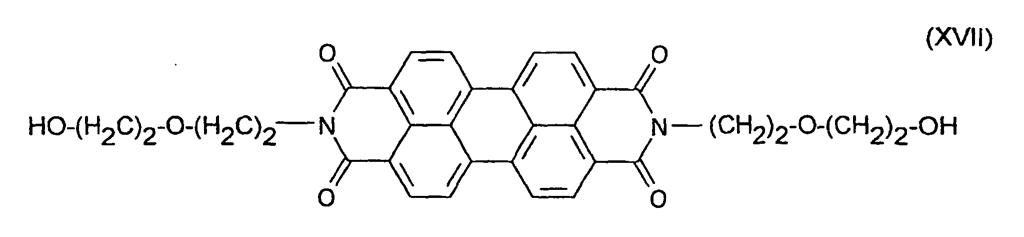

In einem Autoklaven werden 500 Teile Wasser vorgelegt, 181,9 Teile Filterkuchen Perylen-3,4,9,10-tetracarbonsäuredianhydrid 21,5 %ig eingetragen und 42 Teile 2-(2-Aminoethoxy)ethanol zugegeben. Anschließend wird unter Druck auf 150 °C erhitzt und 5 Stunden bei 150 °C gerührt. Nach dem Abkühlen auf 25 °C wird der Pigmentdispergator abgesaugt und mit Wasser neutral gewaschen.
Man erhält 161,7 Teile Presskuchen Pigmentdispergator 38,4 %ig.

| | | |
|---|---|---|
| Analyse | Ber. | 4.9 % N |
| | Gef. | 4.9 % N |

¹H-NMR-Spektrum (D₂SO₄): δ 8,7; 5,25; 4,45 und 4,3 ppm.
Die Lösemittelechtheit des Pigmentdispergators ist sehr gut.

### Beispiel 14a

In einem Rührgefäß werden 366,5 Teile Wasser vorgelegt und unter Rühren 106 Teile Filterkuchen Perylen-3,4,9,10-tetracarbonsäuredianhydrid 21,3 %ig eingetragen. Zu dieser Suspension werden 2,4 Teile einer handelsüblichen 50 %igen wäßrigen Harzseife gegeben und 3,12 Teile Presskuchen Pigmentdispergator 38,4 %ig der Formel XVII, hergestellt gemäß Beispiel 14, eingetragen. Nach Abkühlung auf 0 bis 5 °C werden während 10 Minuten 40,8 Teile einer 40 %igen wäßrigen Monomethylaminlösung zugetropft. Es wird noch 15 Minuten bei 0 bis 5°C gerührt. Dann wird eine Lösung aus 12,8 Teilen Calciumchlorid *2H₂O in 42,5 Teilen Wasser zugetropft und 1 Stunde bei 0 bis 5°C gerührt. Die Suspension wird auf 80 °C erhitzt und 2 Stunden bei 80 °C gerührt. Nach dem Abkühlen auf 60 °C wird bei dieser Temperatur Essigsäure zugetropft, bis ein pH-Wert von 8 erreicht ist. Die erhaltene Pigmentzubereitung wird abgesaugt, mit Wasser chlorionenfrei gewaschen und bei 80 °C im Umluftschrank getrocknet.
Man erhält 25,3 Teile Pigmentzubereitung.
Die Pigmentzubereitung liefert im HS-Lack transparente und farbstarke Lackierungen. Die Rheologie wird mit 3 bis 4 bewertet.

### Beispiel 15

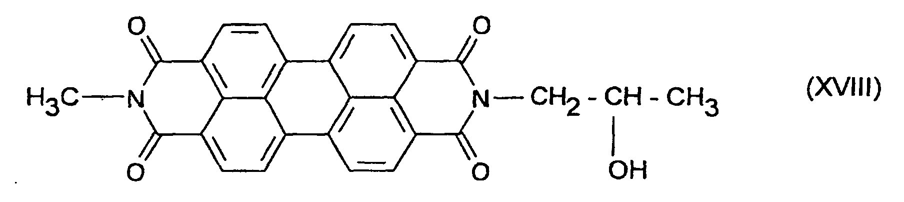

In einem Autoklaven werden 900 Teile Wasser vorgelegt, 40,5 Teile Perylen-3,4,9,10-tetracarbonsäuremonoanhydridmonomethylimid eingetragen und 30 Teile 1-Amino-2-hydroxypropan zugegeben. Anschließend wird unter Druck auf 150 °C erhitzt und 5 Stunden bei 150 °C gerührt. Nach dem Abkühlen auf 25 °C wird der Pigmentdispergator abgesaugt und mit Wasser neutral gewaschen.
Man erhält 117,7 Teile Presskuchen Pigmentdispergator 38 %ig. Ein Teil wird für die Analyse und für mechanische Mischungen bei 80 °C getrocknet.

| | | |
|---|---|---|
| Analyse | Ber. | 6.1 % N |
| | Gef. | 6.1 % N |

¹H-NMR-Spektrum (D₂SO₄): δ 8,7; 5,7; 4,5; 4,0; 3,5 und 1,5 ppm.
Die Lösemittelechtheit des Pigmentdispergators ist sehr gut.

### Beispiel 15a

10 Teile Pigment (C.I. Pigment Red 179), hergestellt gemäß Beispiel 2a, werden mit 0,45 Teilen Pigmentdispergator der Formel XVIII, hergestellt gemäß Beispiel 15, mechanisch gemischt.

Man erhält eine Pigmentzubereitung, die im HS-Lack transparente und farbstarke Lackierungen liefert.

### Beispiel 16

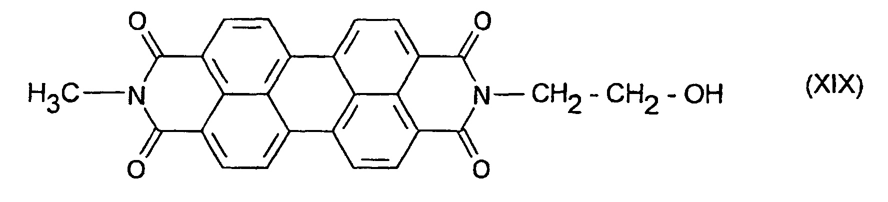

In einem Autoklaven werden 300 Teile Wasser vorgelegt, 22,7 Teile Perylen-3,4,9,10-tetracarbonsäuremonoanhydridmonohydroxyethylimid eingetragen und 15,5 Teile einer 40 %igen wäßrigen Monomethylaminlösung zugegeben. Anschließend wird unter Druck auf 150 °C erhitzt und 5 Stunden bei 150 °C gerührt. Nach dem Abkühlen auf 25 °C wird der Pigmentdispergator abgesaugt und mit Wasser neutral gewaschen.
Man erhält 66,2 Teile Presskuchen Pigmentdispergator 35,1 %ig. Ein Teil wird für die Analyse und für mechanische Mischungen bei 80 °C getrocknet.

| | | |
|---|---|---|
| Analyse | Ber. | 72.3 % C |
| | Gef. | 72.1 % C |

¹H-NMR-Spektrum (D₂SO₄): δ 8,7; 5,16; 4,5; 4,41 und 3,48 ppm.
Die Lösemittelechtheit des Pigmentdispergators ist sehr gut.

### Beispiel 16a

20 Teile Pigment (C.I. Pigment Red 179), hergestellt gemäß Beispiel 2a, werden mit 1 Teil Pigmentdispergator der Formel XIX, hergestellt gemäß Beispiel 16, mechanisch gemischt.
Man erhält eine Pigmentzubereitung, die im HS-Lack transparente und farbstarke Lackierungen liefert.

### Beispiel 17

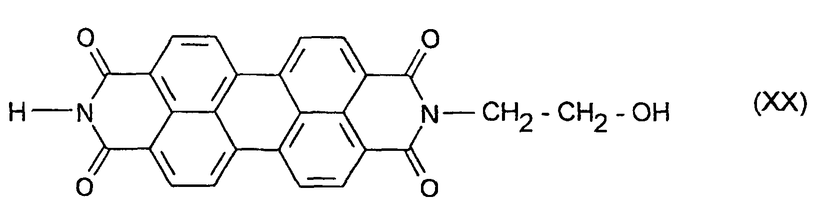

In einem Autoklaven werden 300 Teile Wasser vorgelegt, 22,7 Teile Perylen-3,4,9,10-tetracarbonsäuremonoanhydridmonohydroxyethylimid eingetragen und 13,2 Teile einer 25 %igen wäßrigen Ammoniaklösung zugegeben. Anschließend wird unter Druck auf 150 °C erhitzt und 5 Stunden bei 150 °C gerührt. Nach dem Abkühlen auf 25 °C wird der Pigmentdispergator abgesaugt und mit Wasser neutral gewaschen.

Man erhält 68,7 Teile Presskuchen Pigmentdispergator 32,2 %ig. Ein Teil wird für die Analyse bei 80 °C getrocknet.

| | | |
|---|---|---|
| Analyse | Ber. | 71.9 % C |
| | Gef. | 71.8 % C |

¹H-NMR-Spektrum (D₂SO₄): δ 8.7, 5.2, 4.53 und 4.45 ppm.
Die Lösemittelechtheit des Pigmentdispergators ist sehr gut.

### Beispiel 17a

In einem Rührgefäß werden 363,5 Teile Wasser vorgelegt und unter Rühren 108,9 Teile Filterkuchen Perylen-3,4,9,10-tetracarbonsäuredianhydrid 20,7 %ig eingetragen. Zu dieser Suspension werden 2,4 Teile einer handelsüblichen 50 %igen wäßrigen Harzseife gegeben und 3,72 Teile Presskuchen Pigmentdispergator 32,2 %ig der Formel XX, hergestellt gemäß Beispiel 17, eingetragen. Nach Abkühlung auf 0 bis 5 °C werden während 10 Minuten 40,8 Teile einer 40 %igen wäßrigen Monomethylaminlösung zugetropft. Es wird noch 15 Minuten bei 0 bis 5°C gerührt. Dann wird eine Lösung aus 12,8 Teilen Calciumchlorid *2H₂O in 42,5 Teilen Wasser zugetropft und 1 Stunde bei 0 bis 5°C gerührt. Die Suspension wird auf 80 °C erhitzt und 2 Stunden bei 80 °C gerührt. Nach dem Abkühlen auf 60 °C wird bei dieser Temperatur Essigsäure zugetropft, bis ein pH-Wert von 8 erreicht ist. Die erhaltene Pigmentzubereitung wird abgesaugt, mit Wasser chlorionenfrei gewaschen und bei 80 °C im Umluftschrank getrocknet.
Man erhält 27,4 Teile Pigmentzubereitung.
Die Pigmentzubereitung liefert im HS-Lack transparente und farbstarke Lackierungen.

### Beispiel 18

In einem Rührgefäß werden 400 Teile o-Dichlorbenzol vorgelegt, 40,5 Teile Perylen-3,4,9,10-tetracarbonsäuremonoanhydridmonomethylimid eingetragen und 52,4 Teile 3,3'-Iminobispropylamin zugegeben. Anschließend wird auf 150 °C erhitzt und 5 Stunden bei 150 °C gerührt. Nach dem Abkühlen auf 25 °C wird der Pigmentdispergator abgesaugt und mit o-Dichlorbenzol bis zum Klarlauf gewaschen. Der Presskuchen wird in Wasser angerührt und restliches o-Dichlorbenzol mit Wasserdampf destillativ entfernt. Der Pigmentdispergator wird abgesaugt, mit Wasser gewaschen und bei 80 °C getrocknet.
Man erhält 40,1 Teile Pigmentdispergator der Formel IX.

| | | |
|---|---|---|
| Analyse | Ber. | 72 % C |
| | Gef. | 73 % C |

Das ¹H-NMR-Spektrum stimmt mit der oben angegebenen Strukturformel überein, die Signale liegen bei einer Verschiebung von 8.7 ppm, 6.1 ppm, 5.7 ppm, 4.2 ppm, 3.5 ppm, 3.0 ppm, 2.8 ppm, 2.0 ppm und 1.8 ppm.

### Beispiel 18a

10 Teile Pigment (C.I. Pigment Red 179), hergestellt gemäß Beispiel 2a, werden mit 0,45 Teilen Pigmentdispergator der Formel IX, hergestellt gemäß Beispiel 18, mechanisch gemischt.

Man erhält eine Pigmentzubereitung, die im HS-Lack transparente und farbstarke Lackierungen liefert. Die Rheologie wird mit 4 bis 5 bewertet und die Glanzmessung ergibt den Wert 73. Die Metalliclackierung ist farbstark und brillant.

### Beispiel 19

In einem Rührgefäß werden 300 Teile 3,3'-Iminobispropylamin vorgelegt und 30 Teile Perylen-3,4,9,10-tetracarbonsäuremonoanhydridmonomethylimid eingetragen. Anschließend wird auf 140 °C erhitzt und 2 Stunden bei 140 °C gerührt. Nach dem Abkühlen auf 25 °C werden 600 Teil Wasser zugegeben, der Pigmentdispergator abgesaugt und mit Wasser gewaschen. Der Filterkuchen wird in 800 Teile 1 %iger Kalilauge eingetragen, anschließend wird auf 90 °C erhitzt und 1 Stunde bei 90 °C gerührt. Danach wird bei 90 °C abgesaugt und mit 1 %iger Kalilauge bis zum farblosen Ablauf gewaschen. Anschließend wird der Pigmentdispergator mit Wasser neutral gewaschen und bei 80 °C getrocknet. Man erhält 33,8 Teile Pigmentdispergator der Formel IX.

| | | |
|---|---|---|
| Analyse | Ber. | 72 % C |
| | Gef. | 72 % C |

Das ¹H-NMR-Spektrum stimmt mit der oben angegebenen Strukturformel überein, die Signale liegen bei einer Verschiebung δ von 8.7 ppm, 6.1 ppm, 5.7 ppm, 4.2 ppm, 3.5 ppm, 3.0 ppm, 2.8 ppm, 2.0 ppm und 1.8 ppm.

### Beispiel 19a

30 Teile Pigment (C.I. Pigment Red 179), hergestellt gemäß Beispiel 2a, werden mit 1,35 Teilen Pigmentdispergator der Formel IX, hergestellt gemäß Beispiel 19, mechanisch gemischt.
Man erhält eine Pigmentzubereitung, die im HS-Lack transparente und farbstarke Lackierungen liefert. Die Rheologie wird mit 4 bis 5 bewertet und die Glanzmessung ergibt den Wert 67. Die Metalliclackierung ist farbstark und brillant.

### Beispiel 20

10 Teile eines handelsüblichen Pigments (C.I. Pigment Red 179), das aus 1,8-Naphthalimid mittels einer alkalischen Schmelze mit anschließender Methylierung hergestellt wurde, werden mit 0,5 Teilen Pigmentdispergator der Formel IX, hergestellt gemäß Beispiel 2, mechanisch gemischt.
Mit der erhaltenen Pigmentzubereitung wird im HS-Lack eine Lackierung hergestellt, ebenso wird mit dem für die Zubereitung verwendeten handelsüblichen Pigment (C.I. Pigment Red 179) im HS-Lack eine Lackierung zubereitet. Der Vergleich der Lackierungen ergibt, daß die Transparenz durch den Einsatz des Pigmentdispergators merklich verbessert wird und die Rheologie von 3 auf 4 bis 5 gesteigert wird. Die Viskosität ist ohne Einsatz des Pigmentdispergators so hoch, daß sie mit dem Viskospatel nicht meßbar ist. Durch den Einsatz des Pigmentdispergators beträgt sie 1,6 s. Der Wert der Glanzmessung wird von 32 auf 76 erhöht.

### Beispiel 21 (Vergleichsbeispiel)

Der Pigmentdispergator der Formel XII wird gemäß US-PS 4,762,569, Beispiel 1 hergestellt.

| | | |
|---|---|---|
| Analyse | Ber. | 9,1 % N |
| | Gef. | 8,8 % N |

¹H-NMR-Spektrum (D₂SO₄): δ 8,7; 4,1; 2,87; 2,8; 1,9 und 0,9 ppm.

### Beispiel 21a (Vergleichsbeispiel)

30 Teile Pigment (C.I. Pigment Red 179), hergestellt gemäß Beispiel 2a, werden mit 1,35 Teilen Pigmentdispergator der Formel XII, hergestellt gemäß Beispiel 21, mechanisch gemischt.

Diese Pigmentzubereitung liefert im HS-Lack eine Lackierung, deren Rheologie im Vergleich zu der Pigmentzubereitung des Beispiels 7 (statt 1,35 Teilen Pigmentdispergator der Formel XII, hergestellt gemäß Beispiel 21, wurden 1,35 Teile Pigmentdispergator der Formel IX, hergestellt gemäß Beispiel 2, zur mechanischen Mischung verwendet) deutlich schlechter ist. Die Lösemittelechtheit ist ungenügend und damit der Lösemittelechtheit der Pigmentzubereitung des Beispiels 7 deutlich unterlegen.

### Beispiel 22

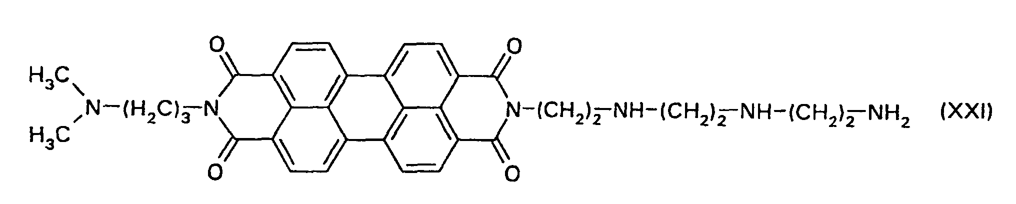

In einem Autoklaven werden 900 Teile Wasser vorgelegt, 44,8 Teile Perylen-3,4,9,10-tetracarbonsäuremonoanhydridmono(dimethylaminopropyl)imid eingetragen und 58,4 Teile Triethylentetramin zugegeben. Anschließend wird unter Druck auf 150°C erhitzt und 5 Stunden bei 150°C gerührt. Nach dem Abkühlen auf 25°C wird der Pigmentdispergator abgesaugt und mit Wasser neutral gewaschen. Man erhält 392,9 Teile Presskuchen Pigmentdispergator 12,4%ig. Ein Teil wird für die Analyse und für mechanische Mischungen bei 80°C getrocknet

| | | |
|---|---|---|
| Analyse | Ber. | 69,5 % C |
| | Gef. | 69,3 % C |

¹H-NMR-Spektrum (D₂SO₄): δ 8.7; 6.6; 6.9; 4.4; 4.1; 3.3; 2.9; 2.5 und 1.9 ppm.

### Beispiel 22a

30 Teile Pigment (C.I. Pigment Red 179), hergestellt gemäß Beispiel 2a, werden mit 1,35 Teilen Pigmentdispergator der Formel XXI, hergestellt gemäß Beispiel 22, mechanisch gemischt.
Man erhält eine Pigmentzubereitung, die im HS-Lack transparente und farbstarke Lackierungen liefert. Die Rheologie wird mit 4 bewertet. Die Metalliclackierung ist farbstark und brillant.

### Beispiel 23

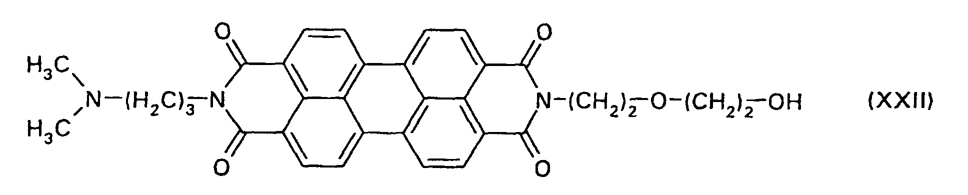

In einem Autoklaven werden 900 Teile Wasser vorgelegt, 44,8 Teile Perylen-3,4,9,10-tetracarbonsäuremonoanhydridmono(dimethylaminopropyl)imid eingetragen und 42 Teile 2-(2-Aminoethoxy)ethanol zugegeben. Anschließend wird unter Druck auf 150°C erhitzt und 5 Stunden bei 150°C gerührt. Nach dem Abkühlen auf 25°C wird der Pigmentdispergator abgesaugt und mit Wasser neutral gewaschen.
Man erhält 129,3 Teile Presskuchen Pigmentdispergator 37,3%ig. Ein Teil wird für die Analyse und für mechanische Mischungen bei 80°C getrocknet

| | | |
|---|---|---|
| Analyse | Ber. | 7,5 % N |
| | Gef. | 7,4 % N |

¹H-NMR-Spektrum (D₂SO₄): δ 8.7; 5.2; 4.4; 4.2; 4.1; 2.9; 2.5 und 2.0 ppm.

### Beispiel 23a

30 Teile Pigment (C.I. Pigment Red 179), hergestellt gemäß Beispiel 2a, werden mit 1,35 Teilen Pigmentdispergator der Formel XXII, hergestellt gemäß Beispiel 23, mechanisch gemischt.
Man erhält eine Pigmentzubereitung, die im HS-Lack transparente und farbstarke Lackierungen liefert. Die Rheologie wird mit 4 bewertet. Die Metalliclackierung ist farbstark und brillant. Die Glanzmessung ergibt den Wert 74.

### Beispiel 24

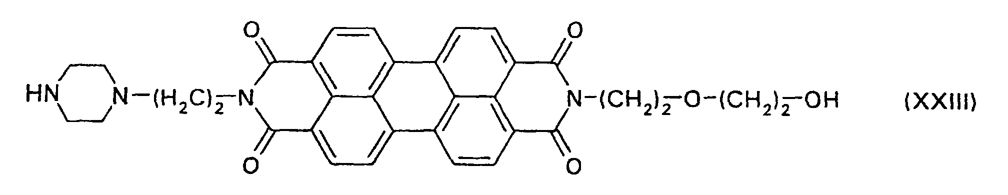

In einem Autoklaven werden 900 Teile Wasser vorgelegt, 47,9 Teile Perylen-3,4,9,10-tetracarbonsäuremonoanhydrid-mono(2-(2-hydroxyethoxy)ethyl)imid eingetragen und 51,6 Teile N-(2-Aminoethyl)piperazin zugegeben. Anschließend wird unter Druck auf 150°C erhitzt und 5 Stunden bei 150°C gerührt. Nach dem Abkühlen auf 90°C wird mit Essigsäure der pH 8-8,5 eingestellt und der Pigmentdispergator abgesaugt und mit Wasser neutral gewaschen.
Man erhält 215,4 Teile Presskuchen Pigmentdispergator 22,7%ig. Ein Teil wird für die Analyse und für mechanische Mischungen bei 80°C getrocknet

| | | |
|---|---|---|
| Analyse | Ber. | 9,5 % N |
| | Gef. | 10,0 % N |

¹H-NMR-Spektrum (D₂SO₄): δ 8.7; 6.6; 6.4; 5.2; 4.4; 4.2; 3.7; 3.5; und 3.2 ppm.

### Beispiel 24a

30 Teile Pigment (C.I. Pigment Red 179), hergestellt gemäß Beispiel 2a, werden mit 1,35 Teilen Pigmentdispergator der Formel XXIII, hergestellt gemäß Beispiel 24, mechanisch gemischt.
Man erhält eine Pigmentzubereitung, die im HS-Lack transparente und farbstarke Lackierungen liefert. Die Rheologie wird mit 4 bis 5 bewertet. Die Metalliclackierung ist farbstark und brillant. Die Glanzmessung ergibt den Wert 64.

## Patentansprüche

1. Perylenverbindung der allgemeinen Formel (I) 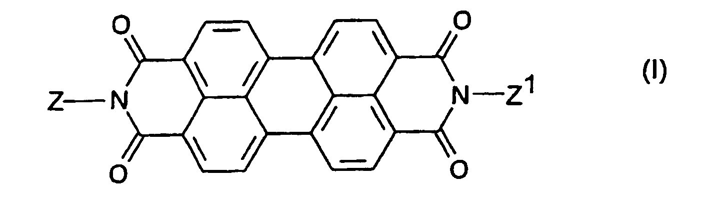 worin
Z¹ ein Rest der Formel (Ia) ist,
- [X- Y]_{q} - [X¹ - Y¹]ᵣ - [X² - NH]ₛ H (Ia)
worin
X, X¹ und X² gleich oder verschieden sind und einen verzweigten oder unverzweigten C₂-C₆-Alkylenrest oder einen C₅-C₇-Cycloalkylenrest bedeuten, der durch 1 bis 4 C₁-C₄-Alkylreste, Hydroxyreste, Hydroxyalkylreste mit 1 bis 4 Kohlenstoffatomen und/oder durch 1 bis 2 weitere C₅-C₇-Cycloalkylreste substituiert sein kann;
Y eine NH-, -O-, 1,4-Piperazindiyl- oder N-(C₁-C₆-alkyl)-Gruppe,
Y¹ eine -NH- oder -O- Gruppe,
q eine Zahl von 1 bis 6,
r und s unabhängig voneinander eine Zahl von 0 bis 6, wobei r und s nicht gleichzeitig Null sind; und wobei s von Null verschieden ist, wenn Y¹ die Bedeutung -O- hat;
Z die Bedeutung Z¹, Z² oder Z³ hat, worin Z² ein Rest der Formel (Ib) ist,
- [X - O]_{q1} - [X¹ - O]_{q} H (Ib)
worin
q1 eine Zahl von 0 bis 6,
und Z³ Wasserstoff, Hydroxy, Amino oder C₁-C₈-Alkyl ist, wobei die Alkylgruppe durch 1 bis 4 Substituenten aus der Gruppe Cl, Br, CN, OH,C₆H₅, Carbamoyl, C₁-C₄-Acyl, C₁-C₄-Alkoxy und NR²R³ substituiert sein kann, oder perfluoriert oder teilfluoriert ist, wobei
R² und R³ unabhängig voneinander ein Wasserstoffatom, eine substituierte oder unsubstituierte, oder teil- oder perfluorierte Alkylgruppe mit 1 bis 20 Kohlenstoffatomen, oder eine substituierte oder unsubstituierte, oder teil- oder perfluorierte Alkenylgruppe mit 2 bis 20 Kohlenstoffatomen darstellen, wobei die Substituenten Hydroxy, Phenyl, Cyano, Chlor, Brom, C₂-C₄-Acyl oder C₁-C₄-Alkoxy sein können, oder R² und R³ zusammen mit dem N-Atom einen, Imidoxolyl-Piperidinyl-, Morpholinyl-, Piperolinyl-, Pyrrolyl-, Pyrrolidinyl-, Pyroxolyl- oder Piperazinyl-Ring bilden,

2. Perylenverbindung nach Anspruch 1, **dadurch gekennzeichnet, daß** Z die Bedeutung Z¹, Z² oder Z³ hat, worin Z² ein Rest der Formel (Ib) ist
- [X - O]_{q1} - [X¹ - O]_{q} H (Ib)
und Z³ Wasserstoff, Hydroxy, Amino oder C₁-C₈-Alkyl ist, wobei die Alkylgruppe durch 1 bis 4 Substituenten aus der Gruppe Cl, Br, CN, OH,C₆H₅, Carbamoyl, C₁-C₄-Acyl und C₁-C₄-Alkoxy substituiert sein kann oder perfluoriert oder teilfluoriert ist.

3. Perylenverbindung nach Anspruch 1 oder 2, **dadurch gekennzeichnet, daß** X, X¹ und X² ein C₂-C₄-Alkylenrest oder Cyclohexylen sind.

4. Perylenverbindung nach mindestens einem der Ansprüche 1 bis 3, **dadurch gekennzeichnet, daß** Z¹ eine der Bedeutungen
-(CH₂)₃-NH-(CH₂)₃-NH₂, -(CH₂)₂-NH-(CH₂)₂-NH₂, -(CH₂)₃-NH-(CH₂)₂-NH-(CH₂)₃-NH₂, 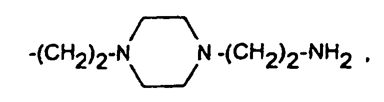 -(CH₂)₃-N(CH₃)-(CH₂)₃-NH₂, -(CH₂)₃-O-(CH₂)₂-O-(CH₂)₃-NH₂, -(CH₂)₃-O-(CH₂)₃-O-(CH₂)₃-NH₂, -(CH₂)₂-NH-(CH₂)₃-NH₂, -(CH₂)₃-NH-(CH₂)₂-NH₂, -(CH₂)₂-NH-(CH₂)₂-NH-(CH₂)₂-NH₂, -(CH₂-CH₂-NH)₄-H, -(CH₂-CH₂-NH)₅-H oder -(CH₂)₃-O-(CH₂)₄-O-(CH₂)₃-NH₂ hat.

5. Perylenverbindung nach mindestens einem der Ansprüche 1 bis 4, **dadurch gekennzeichnet, daß** Z³ Wasserstoff, Benzyl, C₁-C₆-Alkyl oder ein durch 1 bis 2 Substituenten aus der Gruppe Hydroxy, Acetyl, Methoxy und Ethoxy substituiertes C₂-C₆-Alkyl, vorzugsweise Wasserstoff, Methyl, Ethyl, Propyl, Butyl, Benzyl, Hydroxyethyl, Hydroxypropyl oder Methoxypropyl ist.

6. Perylenverbindung nach Anspruch 5, **dadurch gekennzeichnet, daß** Z³ ein Rest der Gruppe -(CH₂)ₙ-NR²R³ ist, worin n eine Zahl von 1 bis 6, vorzugsweise 2 bis 4, und
R² und R³ unabhängig voneinander ein Wasserstoffatom, eine C₁-C₆-Alkylgruppe, eine durch 1 bis 2 Substituenten aus der Gruppe Hydroxy, Acetyl, Methoxy, Ethoxy, Chlor und Brom substituierte C₁-C₆-Alkylgruppe, oder R² und R³ zusammen mit dem angrenzenden N-Atom einen Imidazolyl-, Piperidinyl-, Morpholinyl-, Pipecolinyl-, Pyrrolyl-, Pyrrolidinyl-, Pyrazolyl- oder Piperazinyl-Ring bilden.

7. Perylenverbindung nach anspruch 6, **dadurch gekennzeichnet, daß** n die Zahl 2 oder 3, und
R² und R³ jeweils eine Methyl- oder Ethylgruppe, oder R² und R³ zusammen mit dem angrenzenden Stickstoffatom einen Imidazolyl, Piperazinyl- oder Morpholinylrest bilden.

8. Perylenverbindung nach mindestens einem der Ansprüche 1 bis 7, **dadurch gekennzeichnet, daß** Z² eine der Bedeutungen -CH(CH₂OH)₂ oder -(CH₂)₂-O-(CH₂)₂OH hat.

9. Verfahren zur Herstellung einer Perylenverbindung nach mindestens einem der Ansprüche 1 bis 8 durch Umsetzung von Perylen-3,4,9,10-tetracarbonsäuremonoanhydridmonoimiden der allgemeinen Formel (IIa) 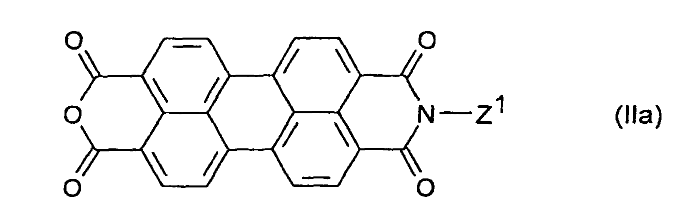 mit einem oder mehreren, vorzugsweise 1 oder 2 Aminen der Formeln (IIIa) oder (IIIb)
Z - NH₂ (IIIa)
Z¹ - NH₂ (IIIb);
oder durch Umsetzung von Perylen-3,4,9,10-tetracarbonsäuremonoanhydridmonoimiden der allgemeinen Formel (lib) 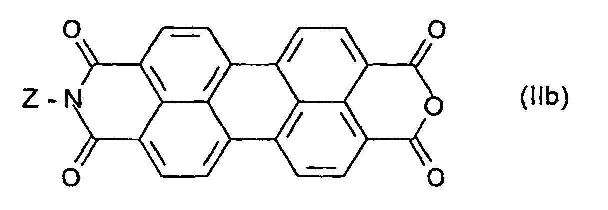 mit einem oder mehreren, vorzugsweise 1 oder 2, Aminen der Formel (IIIb); oder durch Umsetzung von Perylen-3,4,9,10-tetracarbonsäure-dianhydrid der Formel (IIc) 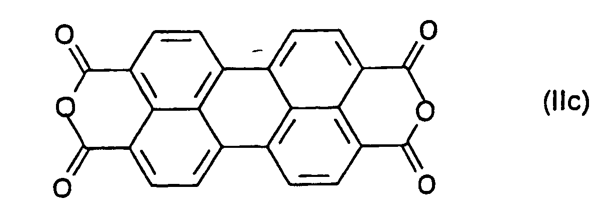 mit einem oder mehreren, vorzugsweise 1 oder 2, Aminen der Formel (IIIb).

10. Pigmentzubereitung, **gekennzeichnet durch** einen Gehalt an
a) mindestens einem organischen Basispigment aus der gruppe der Perylen-, Perinon-, Phthalocyanin-, Dioxazin-, Chinacridon-, Azo-,Anthrachinon-, Aminoanthrachinon-,Thioindigo-, Diketopyrrolopyrrol, Flavanthron-, Indanthron-, Isoindolin-, Isoindolinon-, Anthrapyrimidin-, Pyranthron-, Chinophthalon-, Isoviolanthron-,Triarylcarbonium-, Carbon Black- (Ruß) und Anthanthronpigmente, wobei das Perylenpigment ein Pigment aus der Gruppe C.I. Pigment Red 123, 149, 178, 179 190 und 224 ist, und
b) mindestens einem Pigmentdispergator der allgemeinen Formel (IV), 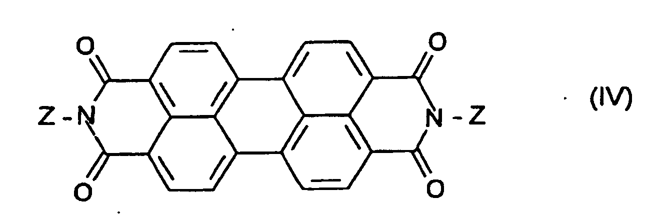
worin die beiden Reste Z gleich oder verschieden sind und die in einem oder mehreren der Ansprüche 1 bis 8 genannten Bedeutungen haben, mit der Maßgabe, daß beide Reste Z nicht gleichzeitig Z³ bedeuten;
ausgenommen eine Pigmentzubereitung, **gekennzeichnet durch** einen Gehalt an
a) mindestens einem organischen Basispigment aus der Klasse der Perylen-, Perinon-, Chinacridon-, Azo-, Benzimidazolon-, Anthrachinon- und AnthanthronPigmente und
b) mindestens einem Pigmentdispergator der Formel (IV), worin einer der beiden Reste Z die Bedeutung C₁-C₈-Alkyl, das **durch** 1 bis 4 Hydroxygruppen substituiert ist, hat, und der andere der beiden Reste Z die Bedeutung -(CH₂)ₙ-NR²R³ hat, worin n eine Zahl von 1 bis 6 ist, und R² und R³ die in Anspruch 1 genannten Bedeutungen haben.

11. Pigmentzubereitung nach Anspruch 10, bestehend im wesentlichen aus
a) 40 bis 99,5 Gew.-%, vorzugsweise 60 bis 99 Gew.-%, mindestens eines Basispigments,
b) 0,5 bis 40 Gew.-%, vorzugsweise 1 bis 20 Gew.-%, mindestens eines, vorzugsweise 1, 2 oder 3, Pigmentdispergators der Formel (IV),
c) 0 bis 20 Gew.-%, vorzugsweise 0,1 bis 15 Gew.-% an oberflächenaktiven Mitteln und
d) 0 bis 20 Gew.-%, vorzugsweise 0,1 bis 10 Gew.-% an anderen üblichen Zusatzstoffen,
wobei die Anteile der jeweiligen Komponenten auf das Gesamtgewicht der Zubereitung (100 Gew.-%) bezogen sind.

12. Pigmentzubereitung, enthaltend
a) mindestens ein organisches Basispigment,
b1) einen Pigmentdispergator der Formel (IX) 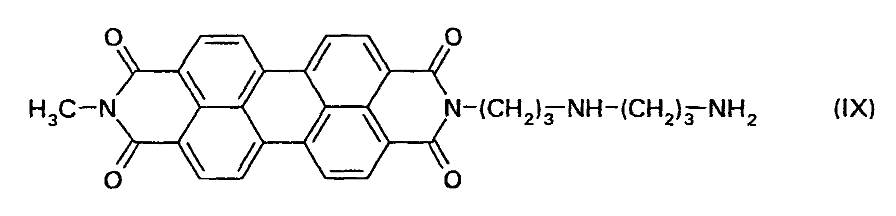 und
b2) einen Pigmentdispergator der Formel (X)

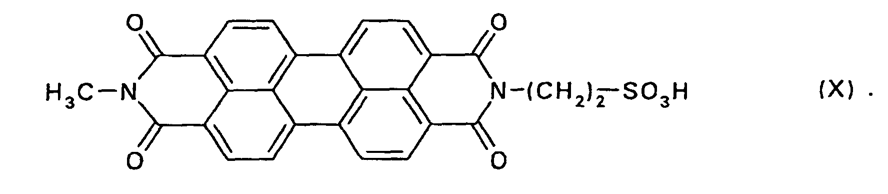

13. Verfahren zur Herstellung einer Pigmentzubereitung nach Anspruch 10, **dadurch gekennzeichnet, daß** man den oder die Pigmentdispergator(en) der Formel (IV) und das oder die Basispigment(e) an einem beliebigen Zeitpunkt ihres Herstellungsprozesses aufeinander einwirken läßt.

14. Verfahren nach Anspruch 13, **dadurch gekennzeichnet, daß** der Pigmentdispergator der Formel (IV) vor, während oder nach der Synthese des Basispigments; vor, während oder nach eines Feinverteilungsprozesses des Basispigments, oder vor, während oder nach einer Lösemittelbehandlung des Basispigments zugegeben wird.

15. Verfahren nach Anspruch 13 oder 14, **dadurch gekennzeichnet, daß** der Pigmentdispergator und/oder das organische Basispigment in Form eines wasserfeuchten Preßkuchens zusammengegeben werden, oder als trockene Granulate oder Pulver miteinander vermischt werden.

16. Verfahren nach Anspruch 13, **dadurch gekennzeichnet, daß** der Pigmentdispergator der Formel (IV) und ein organisches Perylenpigment durch Umsetzung von Perylen-3,4,9,10-tetracarbonsäuremonoanhydridmonoimiden und Perylen-3,4,9,10-tetracarbonsäure-dianhydrid mit demselben Amin der Formel (IIIa) oder (IIIb) gemäß Anspruch 9 als Mischung hergestellt werden.

17. Verwendung der gemäß einem oder mehreren der Ansprüche 10 bis 12 definierten Pigmentzubereitungen als Farbmittel zum Pigmentieren von hochmolekularen organischen Materialien natürlicher oder synthetischer Herkunft in Form plastischer Massen, Schmelzen, Spinnlösungen, Lacken, Anstrichfarben, Druckfarben, elektrophotographischen Tonern, Pulverlacken und Ink-Jet-Tinten.

18. Pigmentpräparation, bestehend im wesentlichen aus mindestens einem organischen Basispigment und mindestens einem Pigmentdispergator der Formel (IV) gemäß Anspruch 10, und einem hochmolekularen organischen Material gemäß Anspruch 18.

## Claims

1. A perylene compound of the formula (I) 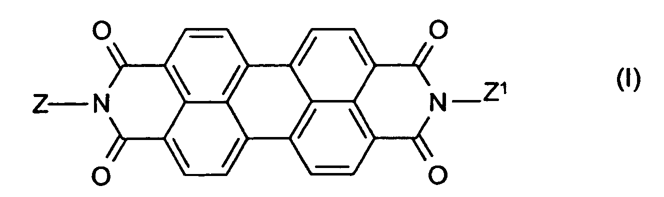 in which
Z¹ is a radical of the formula (Ia)
- [X- Y]_{q} - [X¹ - Y¹]ᵣ - [X² - NH]ₛ H (Ia)
in which
X, X¹ and X² are identical or different and are a branched or unbranched C₂-C₆-alkylene radical or a C₅-C₇-cycloalkylene radical which can be substituted by from 1 to 4 C₁-C₄-alkyl radicals, hydroxyl radicals, hydroxyalkyl radicals having 1 to 4 carbon atoms, and/or by from 1 to 2 further C₅-C₇-cycloalkyl radicals;
Y is an NH-, -O-, 1,4-piperazinediyl or N(C₁-C₆-alkyl) group,
Y¹ is an -NH- or -O- group,
q is a number from 1 to 6,
r and s independently of one another are a number from 0 to 6, but are not simultaneously zero; and s is not zero when Y¹ is -O-;
Z is defined as Z¹, Z² or Z³, where Z² is a radical of the formula (Ib)
- [X - O]_{q1} - [X¹ - O]_{q} H (Ib)
in which
q1 is a number from 0 to 6,
and Z³ is hydrogen, hydroxyl, amino or C₁-C₈-alkyl where the alkyl group can be substituted by from 1 to 4 substituents from the group consisting of Cl, Br, CN, OH, C₆H₅, carbamoyl, C₁-C₄-acyl, C₁-C₄-alkoxy and NR²R³, or is perfluorinated or partly fluorinated, and where
R² and R³ independently of one another are a hydrogen atom, a substituted or unsubstituted or partly fluorinated or perfluorinated alkyl group of 1 to 20 carbon atoms, or a substituted or unsubstituted or partly fluorinated or perfluorinated alkenyl group of 2 to 20 carbon atoms, where the substituents can be hydroxyl, phenyl, cyano, chloro, bromo, C₂-C₄-acyl or C₁-C₄-alkoxy, or
R² and R³, together with the nitrogen atom, form an imidazolyl, piperidinyl, morpholinyl, pipecolinyl, pyrrolyl, pyrrolidinyl, pyrazolyl or piperazinyl ring.

2. A perylene compound as claimed in claim 1, wherein Z is defined as Z¹, Z² or Z³, where Z² is a radical of the formula (Ib)
- [X - O]_{q1} - [X¹ - O]_{q} H (Ib)
and Z³ is hydrogen, hydroxyl, amino or C₁-C₈-alkyl, where the alkyl group can be substituted by from 1 to 4 substituents from the group consisting of Cl, Br, CN, OH,C₆H₅, carbamoyl, C₁-C₄-acyl and C₁-C₄-alkoxy or is perfluorinated or partly fluorinated.

3. A perylene compound as claimed in claim 1 or 2, wherein X, X¹ and X² are a C₂-C₄-alkylene radical or cyclohexylene.

4. A perylene compound as claimed in at least one of claims 1 to 3, wherein Z¹ has one of the definitions -(CH₂)₃-NH-(CH₂)₃-NH₂, -(CH₂)₂-NH-(CH₂)₂-NH₂, -(CH₂)₃-NH-(CH₂)₂-NH-(CH₂)₃-NH₂, 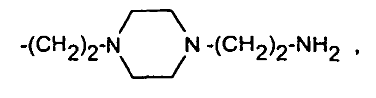 -(CH₂)₃-N(CH₃)-(CH₂)₃-NH₂, -(CH₂)₃-O-(CH₂)₂-O-(CH₂)₃-NH₂, -(CH₂)₃-O-(CH₂)₃-O-(CH₂)₃-NH₂, -(CH₂)₂-NH-(CH₂)₃-NH₂, -(CH₂)₃-NH-(CH₂)₂-NH₂, -(CH₂)₂-NH-(CH₂)₂-NH-(CH₂)₂-NH₂, -(CH₂-CH₂-NH)₄-H, -(CH₂-CH₂-NH)₅-H or -(CH₂)₃-O-(CH₂)₄-O-(CH₂)₃-NH₂.

5. A perylene compound as claimed in at least one of claims 1 to 4, wherein Z³ is hydrogen, benzyl, C₁-C₆-alkyl or a C₂-C₆-alkyl which is substituted by from 1 to 2 substituents from the group consisting of hydroxyl, acetyl, methoxy and ethoxy, and preferably is hydrogen, methyl, ethyl, propyl, butyl, benzyl, hydroxyethyl, hydroxypropyl or methoxypropyl.

6. A perylene compound as claimed in claim 5, wherein Z³ is a radical of the group -(CH₂)ₙ-NR²R³ in which n is a number from 1 to 6, preferably from 2 to 4, and
R² and R³ independently of one another are a hydrogen atom, a C₁-C₆-alkyl group, a C₁-C₆-alkyl group which is substituted by from 1 to 2 substituents from the group consisting of hydroxyl, acetyl, methoxy, ethoxy, chloro and bromo, or R² and R³, together with the adjacent nitrogen atom, form an imidazolyl, piperidinyl, morpholinyl, pipecolinyl, pyrrolyl, pyrrolidinyl, pyrazolyl or piperazinyl ring.

7. A perylene compound as claimed in claim 6, wherein n is the number 2 or 3, and
R² and R³ are each a methyl or ethyl group or R² and R³, together with the adjacent nitrogen atom, form an imidazolyl, piperazinyl or morpholinyl radical.

8. A perylene compound as claimed in at least one of claims 1 to 7, wherein Z² has one of the definitions -CH(CH₂OH)₂ or -(CH₂)₂-O-(CH₂)₂OH.

9. A process for preparing a perylene compound as claimed in at least one of claims 1 to 8 by reacting perylene-3,4,9,10-tetracarboxylic monoanhydride monoimides of the formula (IIa) 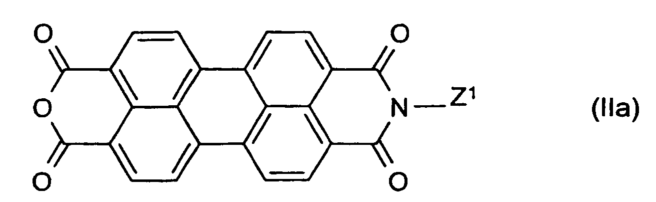 with one or more, preferably 1 or 2, amines of the formula (IIIa) or (IIIb)
Z - NH₂ (IIIa)
Z¹ - NH₂ (IIIb);
or by reacting perylene-3,4,9,10-tetracarboxylic monoanhydride monoimides of the formula (IIb) 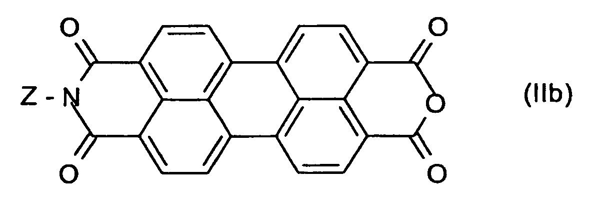 with one or more, preferably 1 or 2, amines of the formula (IIIb);
or by reacting perylene-3.4,9,10-tetracarboxylic dianhydride of the formula (IIc) 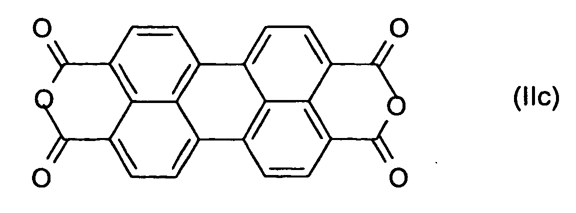 with one or more, preferably 1 or 2, amines of the formula (IIIb).

10. A pigment preparation comprising
a) at least one organic base pigment from the group of the perylene, perinone, phthalocyanine, dioxazine, quinacridone, azo, anthraquinone, aminoanthraquinone, thioindigo, diketopyrrolopyrrole, flavanthrone, indanthrone, isoindoline, isoindolinone, anthrapyrimidine, pyranthrone, quinophthalone, isoviolanthrone, triarylcarbonium, carbon black and anthanthrone pigments, where the perylene pigment is a pigment from the group C.I. Pigment Red 123, 149, 178, 179, 190 and 224, and
b) at least one pigment dispersant of the formula (IV) 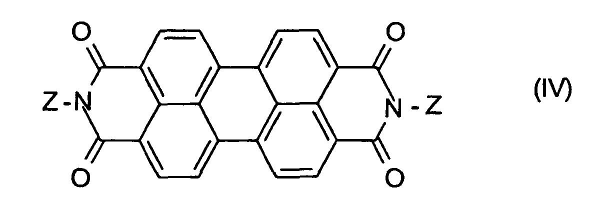
in which the two radicals Z are identical or different and are as defined in one or more of claims 1 to 8 with the proviso that the two radicals Z are not simultaneously Z³; with the exception of a pigment preparation comprising
a) at least one organic base pigment from the class of perylene, perinone, quinacridone, azo, benzimidazolone, anthraquinone and anthanthrone pigments and
b) at least one pigment dispersant of formula (IV) in which one of the two radicals Z has the definition of C₁-C₈-alkyl substituted by 1 to 4 hydroxyl groups and the other of the two radicals Z has the definition -(CH₂)ₙ-NR²R³ in which n is a number from 1 to 6 and R² and R³ are as defined in claim 1.

11. A pigment preparation as claimed in claim 10 consisting essentially of
a) from 40 to 99.5% by weight, preferably from 60 to 99% by weight, of at least one base pigment,
b) from 0.5 to 40% by weight, preferably from 1 to 20% by weight, of at least one, preferably 1, 2 or 3, pigment dispersants of the formula (IV),
c) from 0 to 20% by weight, preferably from 0.1 to 15% by weight, of surface-active agents, and
d) from 0 to 20% by weight, preferably from 0.1 to 10% by weight, of other customary additives,
the proportions of the respective components being based on the overall weight of the preparation (100% by weight).

12. A pigment preparation comprising
a) at least one organic base pigment,
b1) a pigment dispersant of the formula (IX) 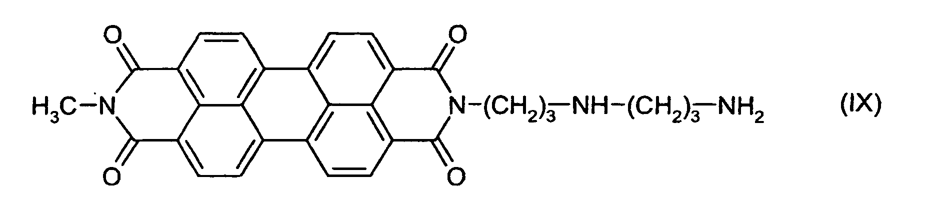 and
b2) a pigment dispersant of the formula (X)

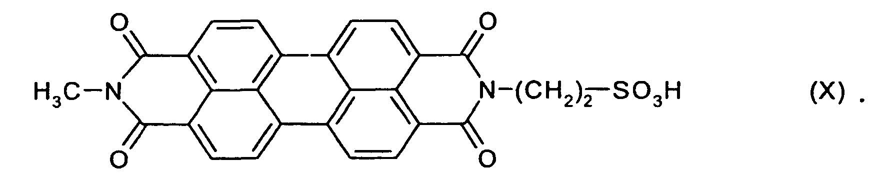

13. A process for preparing a pigment preparation as claimed in claim 10, which comprises allowing the pigment dispersant(s) of the formula (IV) and the base pigment(s) to act on one another at any desired point in time during their preparation process.

14. The process as claimed in claim 13, wherein the pigment dispersant of the formula (IV) is added prior to, during or after the synthesis of the base pigment; prior to, during or after a process of fine division of the base pigment; or prior to, during or after a solvent treatment of the base pigment.

15. The process as claimed in claim 13 or 14, wherein the pigment dispersant and/or the organic base pigment are combined in the form of a water-moist presscake or are mixed with one another as dry granules or powders.

16. The process as claimed in claim 13, wherein the pigment dispersant of the formula (IV) and an organic perylene pigment are prepared by reacting perylene-3,4,9,10-tetracarboxylic monoanhydride monoimides and perylene-3,4,9,10-tetracarboxylic dianhydride with the same amine of the formula (IIIa) or (IIIb), as set forth in claim 9, as a mixture.

17. The use of a pigment preparation defined as set forth in one or more of claims 10 to 12 as a colorant for pigmenting high molecular mass organic materials of natural or synthetic origin in the form of plastic masses, melts, spinning solutions, varnishes, paints, printing inks, electrophotographic toners, powder coating materials or inkjet inks.

18. A prepared pigment formulation consisting essentially of at least one organic base pigment and at least one pigment dispersant of the formula (IV) as set forth in claim 10, and a high molecular mass organic material as set forth in claim 17.

## Revendications

1. Composé pérylène de formule générale (I) 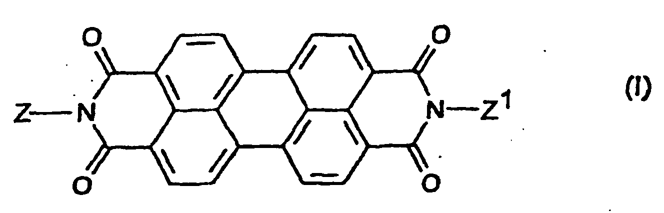 dans laquelle
Z¹ représente un groupe de formule (Ia),
-[X-Y]_{q}-[X¹-Y¹]ᵣ-[X²-NH]ₛH (Ia)
dans laquelle
X, X¹ et X² sont identiques ou différents et représentent un groupe alkylène en C₂ à C₆ ramifié ou non ramifié ou un groupe cycloalkylène en C₅ à C₇, qui peut être substitué par 1 à 4 groupes alkyle en C₁ à C₄, groupes hydroxy, groupes hydroxyalkyle contenant 1 à 4 atomes de carbone et/ou par 1 à 2 autres groupes cycloalkyle en C₅ à C₇ ;
Y représente un groupe NH-, -O-, un groupe 1,4-pipérazindiyle ou N(alkyle en C₁ à C₆),
Y¹ représente un groupe -NH- ou -O-,
q représente un nombre de 1 à 6,
r et s représentent, indépendamment l'un de l'autre, un nombre de 0 à 6, r et s ne valant pas zéro en même temps ; et s ne valant pas zéro lorsque Y¹ représente -O- ;
Z a la signification de Z¹, Z² ou Z³, où Z² représente un groupe de formule (Ib),
-[X-O]_{q1}-[X¹O]_{q}H (Ib)
dans laquelle
q1 représente un nombre de 0 à 6,
et Z³ représente un atome d'hydrogène, un groupe hydroxy, amino ou alkyle en C₁ à C₈, le groupe alkyle pouvant être substitué par 1 à 4 substituants du groupe formé par Cl, Br, CN, OH, C₆H₅, carbamoyle, acyle en C₁ à C₄, alcoxy en C₁ à C₄ et NR²R³, ou étant perfluoré ou partiellement fluoré,
où R² et R³ représentent, indépendamment l'un de l'autre, un atome d'hydrogène, un groupe alkyle contenant 1 à 20 atomes de carbone substitué ou non substitué, ou partiellement fluoré ou perfluoré, ou un groupe alcényle contenant 2 à 20 atomes de carbone substitué ou non substitué, ou partiellement fluoré ou perfluoré, les substituants pouvant être des groupes hydroxy, phényle, cyano, chloro, bromo, acyle en C₂ à C₄ ou alcoxy en C₁ à C₄, ou bien R² et R³ représentent, conjointement avec l'atome d'azote, un noyau imidazolyle, pipéridinyle, morpholinyle, pipécolinyle, pyrrolyle, pyrrolidinyle, pyrazolyle ou pipérazinyle.

2. Composé pérylène selon la revendication 1, **caractérisé en ce que** Z a la signification de Z¹, Z² ou Z³, où Z² représente un groupe de formule (Ib)
-[X-O]_{q1}-[X¹-O]_{q}H (Ib)
et Z³ représente un atome d'hydrogène, un groupe hydroxy, amino ou alkyle en C₁ à C₈, le groupe alkyle pouvant être substitué par 1 à 4 substituants du groupe formé par Cl, Br, CN, OH, C₆H₅, carbamoyle, acyle en C₁ à C₄ et alcoxy en C₁ à C₄, ou étant perfluoré ou partiellement fluoré.

3. Composé pérylène selon la revendication 1 ou 2, **caractérisé en ce que** X, X¹ et X² représentent un groupe alkylène en C₂ à C₄ ou cyclohexylène.

4. Composé pérylène selon au moins l'une des revendications 1 à 3, **caractérisé en ce que** Z¹ a l'une des significations suivantes :
-(CH₂)₃-NH-(CH₂)₃-NH₂, -(CH₂)₂-NH-(CH₂)₂-NH₂, -(CH₂)₃-NH-(CH₂)₂-NH-(CH₂)₃-NH₂, 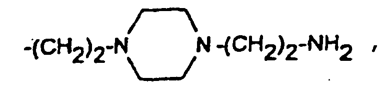 -(CH₂)₃-N(CH₃)-(CH₂)₃-NH₂, -(CH₂)₃-O-(CH₂)₂-O-(CH₂)₃-NH₂, -(CH₂)₃-O-(CH₂)₃-O-(CH₂)₃-NH₂, -(CH₂)₂-NH-(CH₂)₃-NH₂, -(CH₂)₃-NH-(CH₂)₂-NH₂, -(CH₂)₂-NH-(CH₂)₂-NH-(CH₂)₂-NH₂, -(CH₂-CH₂-NH)₄-H, -(CH₂-CH₂-NH)₅-H ou -(CH₂)₃-O-(CH₂)₄-O-(CH₂)₃-NH₂.

5. Composé pérylène selon au moins l'une des revendications 1 à 4, **caractérisé en ce que** Z³ représente un atome d'hydrogène, un groupe benzyle, alkyle en C₁ à C₆ ou un groupe alkyle en C₂ à C₆ substitué par 1 à 2 substituants du groupe formé par les groupes hydroxy, acétyle, méthoxy et éthoxy, de préférence un atome d'hydrogène, un groupe méthyle, éthyle, propyle, butyle, benzyle, hydroxyéthyle, hydroxypropyle ou méthoxypropyle.

6. Composé pérylène selon la revendication 5, **caractérisé en ce que** Z³ représente un radical du groupe -(CH₂)ₙ-NR²R³, dans lequel n représente un nombre de 1 à 6, de préférence de 2 à 4, et
R² et R³ représentent, indépendamment l'un de l'autre, un atome d'hydrogène, un groupe alkyle en C₁ à C₆, un groupe alkyle en C₁ à C₆ substitué par 1 à 2 substituants du groupe formé par les groupes hydroxy, acétyle, méthoxy, éthoxy, chloro et bromo, ou bien R² et R³ représentent, conjointement avec l'atome d'azote adjacent, un noyau imidazolyle, pipéridinyle, morpholinyle, pipécolinyle, pyrrolyle, pyrrolidinyle, pyrazolyle ou pipérazinyle.

7. Composé pérylène selon la revendication 6, **caractérisé en ce que** n vaut 2 ou 3 et
R² et R³ représentent chacun un groupe méthyle ou éthyle, ou bien R² et R³ représentent, conjointement avec l'atome d'azote adjacent, un noyau imidazolyle, pipérazinyle ou morpholinyle.

8. Composé pérylène selon au moins l'une des revendications 1 à 7, **caractérisé en ce que** Z² a l'une des significations suivantes : -CH(CH₂OH)₂ ou -(CH₂)₂-O-(CH₂)₂OH.

9. Procédé de préparation d'un composé pérylène selon au moins l'une des revendications 1 à 8 en faisant réagir des monoimides de monoanhydrides pérylène-3,4,9,10-tétracarboxyliques de formule générale (IIa) 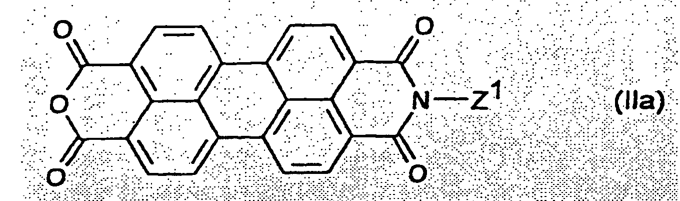 avec une ou plusieurs, de préférence une ou deux amines de formule (IIIa) ou (IIIb)
Z - NH₂ (IIIa)
Z¹ - NH₂ (IIIb);
ou en faisant réagir des monoimides de monoanhydrides pérylène-3,4,9,10-tétracarboxyliques de formule générale (IIb) 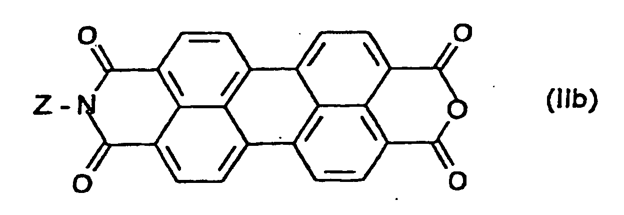 avec une ou plusieurs, de préférence une ou deux amines de formule (IIIb) ; ou en faisant réagir des dianhydrides pérylène-3,4,9,10-tétracarboxyliques de formule générale (IIc) 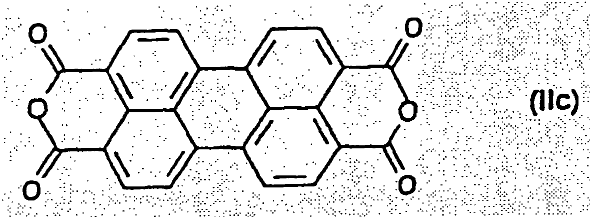 avec une ou plusieurs, de préférence une ou deux amines de formule (IIIb).

10. Préparation de pigment, **caractérisée par** une certaine teneur en
a) au moins un pigment de base organique du groupe formé par les pigments pérylène, périnone, phtalocyanine, dioxazine, quinacridone, azoïque, anthraquinone, aminoanthraquinone, thioindigo, dicétopyrrolopyrrole, flavanthrone, indanthrone, isoindoline, isoindolinone, anthrapyrimidine, pyranthrone, quinophtalone, isoviolanthrone, triarylcarbonium, noir de carbone et anthranthone, le pigment pérylène étant un pigment du groupe formé par C.I. Pigment Red 123, 149, 178, 179, 190 et 224, et
b) au moins un dispersant de pigment de formule générale (IV), 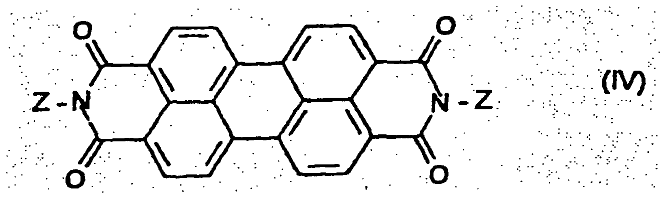
dans laquelle les deux groupes Z sont identiques ou différents et ont les significations mentionnées dans une ou plusieurs des revendications 1 à 8, à condition que les deux groupes Z n'aient pas la signification de Z³ en même temps ;
excepté une préparation de pigment, **caractérisée par** une certaine teneur en
a) au moins un pigment de base organique du groupe formé par les pigments pérylène, périnone, quinacridone, azoïque, benzimidazolone, anthraquinone et anthanthrone et
b) au moins un dispersant de pigment de formule (IV), dans laquelle l'un des deux groupes Z représente un groupe alkyle en C₁ à C₈, qui est substitué par 1 à 4 groupes hydroxy, et l'autre des deux groupes Z représente un groupe -(CH₂)ₙ-NR²R³, dans lequel n représente un nombre de 1 à 6, et R² et R³ ont les significations mentionnées dans la revendication 1.

11. Préparation de pigment selon la revendication 10, essentiellement composée de
a) 40% à 99,5% en poids, de préférence 60% à 99% en poids d'au moins un pigment de base,
b) 0,5% à 40% en poids, de préférence 1% à 20% en poids, d'au moins un, de préférence 1, 2 ou 3, dispersant de pigment de formule (IV),
c) 0% à 20% en poids, de préférence 0,1% à 15% en poids de tensio-actifs,
d) 0% à 20% en poids, de préférence 0,1% à 10% en poids d'autres adjuvants habituels,
les proportions des composants se rapportant à chaque fois au poids total de la préparation (100% en poids).

12. Préparation de pigment, contenant
a) au moins un pigment de base organique,
b1) un dispersant de pigment de formule (IX) 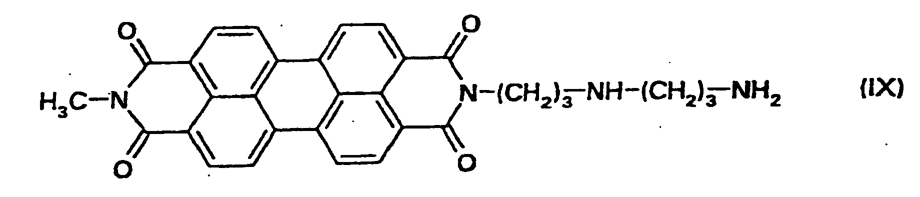 et
b2) un dispersant de pigment de formule (X)

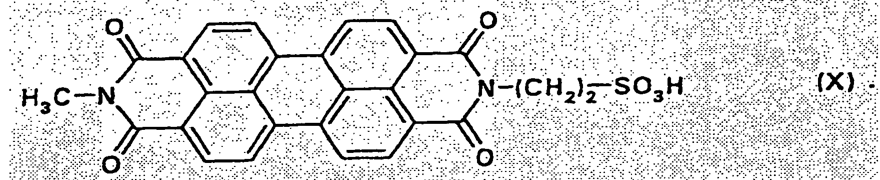

13. Procédé de production d'une préparation de pigment selon la revendication 10, **caractérisé en ce que** l'on fait agir le ou les dispersants de pigment de formule (IV) et le ou les pigments de base les uns avec les autres à tout moment souhaité de leur processus de production.

14. Procédé selon la revendication 13, **caractérisé en ce que** le dispersant de pigment de formule (IV) est ajouté avant, pendant ou après la synthèse du pigment de base ; avant, pendant ou après un procédé de division en fines particules du pigment de base ou bien avant, pendant ou après un traitement au solvant du pigment de base.

15. Procédé selon la revendication 13 ou 14, **caractérisé en ce que** l'on ajoute le dispersant de pigment et/ou le pigment de base organique sous la forme d'un gâteau de filtre-presse humide d'eau ou **en ce qu'**on les mélange les uns avec les autres sous forme de granulés secs ou de poudre sèche.

16. Procédé selon la revendication 13, **caractérisé en ce que** l'on prépare le dispersant de pigment de formule (IV) et un pigment pérylène organique sous forme de mélange en faisant réagir des monoimides de monoanhydrides pérylène-3,4,9,10-tétracarboxyliques et des dianhydrides pérylène-3,4,9,10-tétracarboxyliques avec la même amine de formule (IIIa) ou (IIIb) selon la revendication 9.

17. Utilisation des préparations de pigments définies selon l'une ou plusieurs des revendications 10 à 12, en tant que colorant pour la pigmentation de matières organiques à haute masse moléculaire d'origine naturelle ou synthétique sous la forme de matières plastiques, masses fondues, solutions de filage, vernis, peintures, encres d'impression, toners électrophotographiques, vernis en poudre et encres pour impression par jet d'encre.

18. Préparation de pigment, essentiellement composée d'au moins un pigment de base organique et d'au moins un dispersant de pigment de formule (IV) selon la revendication 10, et d'une matière organique à haute masse moléculaire selon la revendication 17.
